# EUROPEAN PATENT APPLICATION

(11) **EP 3 617 306 A1**
(43) Date of publication of application: **04.03.2020**
(21) Application number: 18789904.2
(22) Date of filing: 25.04.2018
(51) Int. Cl.: C12N 5/0775, A61K 35/28, C12Q 1/6851, G01N 33/53, C12N 15/09

(54) **METHOD FOR PRODUCING MESENCHYMAL STEM CELLS, THERAPEUTIC EFFECT MARKER OF MESENCHYMAL STEM CELLS, METHOD FOR DETERMINING THERAPEUTIC EFFECTS OF MESENCHYMAL STEM CELLS, AND CELLULAR PREPARATION CONTAINING MESENCHYMAL STEM CELLS**

(30) Priority: 25.04.2017 JP 2017086514; 03.05.2017 JP 2017092030
(71) Applicant: Sapporo Medical University, Sapporo-shi, Hokkaido 060-8556 (JP)
(72) Inventor: CHIKENJI, Takako, Sapporo-shi Hokkaido 060-8556 (JP); FUJIMIYA, Mineko, Sapporo-shi Hokkaido 060-8556 (JP); SAITO, Yuki, Sapporo-shi Hokkaido 060-8556 (JP); NAKANO, Masako, Sapporo-shi Hokkaido 060-8556 (JP); OTANI, Miho, Sapporo-shi Hokkaido 060-8556 (JP); MIZUE, Yuka, Sapporo-shi Hokkaido 060-8556 (JP); MATSUMURA, Takashi, Sapporo-shi Hokkaido 060-8556 (JP); KAMIYA, Kozue, Sapporo-shi Hokkaido 060-8556 (JP)
(74) Representative: Wasner, Marita
(86) International application number: PCT/JP2018/016883
(87) International publication number: WO 2018/199194

(57) **Abstract**

[Problem] To provide a cell preparation including mesenchymal stem cells (MSCs) having a high therapeutic effect.

[Solution] A method for producing activated mesenchymal stem cells, including a step of culturing MSCs in a medium containing an activator that includes an extract from a mammalian fetal appendage as an active ingredient, using a cell culture carrier having a three-dimensional structure formed of a fiber is provided. A marker for a therapeutic effect of MSCs selected from the group consisting of p16^{ink4a}, p14^{ARF}' CDK4, CDK6, RB, and CD47, a method for determining a therapeutic effect using the maker, a method for determining suitability of MSCs to be treated with a treatment for enhancing a therapeutic effect of MSCs, a cell preparation including MSCs, and a method for producing the same are also provided.

SEQUENCE LISTING

<110>  SAPPORO MEDICAL UNIVERSITY

<120>  METHOD FOR PRODUCING MESENCHYMAL STEM CELL, MARKER FOR
       THERAPEUTIC EFFECT OF MESENCHYMAL STEM CELL, METHOD FOR
       DETERMINING THERAPEUTIC EFFECT OF MESENCHYMAL STEM CELL, AND CELL
        PREPARATION CONTAINING MESENCHYMAL STEM CELL

<130>  P16SM01WO

<160>  34

<170>  PatentIn version 3.5

<210>  1
<211>  18
<212>  DNA
<213>  Artificial Sequence

<220>
<223>  OCT4 Forward Primer

<400>  1
gacaggggag gggaggag                                                    18


<210>  2
<211>  21
<212>  DNA
<213>  Artificial Sequence

<220>
<223>  OCT4 Reverse Primer

<400>  2
cttccctcca accagttgcc c                                                21


<210>  3
<211>  21
<212>  DNA
<213>  Artificial Sequence

<220>
<223>  NANOG Forward Primer

<400>  3
tggacactgg ctgaatcctt c                                                21


<210>  4
<211>  21
<212>  DNA
<213>  Artificial Sequence

<220>
<223>  NANOG Reverse Primer

<400>  4
cgttgattag gctccaacca t                                                21

<210>  5
<211>  20
<212>  DNA
<213>  Artificial Sequence

<220>
<223>  SOX2 Forward Primer

<400>  5
gggaaatggg aggggtgcaa                                                    20


<210>  6
<211>  20
<212>  DNA
<213>  Artificial Sequence

<220>
<223>  SOX2 Reverse Primer

<400>  6
ttgcgtgagt gtggatggga                                                    20


<210>  7
<211>  22
<212>  DNA
<213>  Artificial Sequence

<220>
<223>  DNMT1 Forward Primer

<400>  7
cgtaaagaag aattatccga gg                                                 22


<210>  8
<211>  21
<212>  DNA
<213>  Artificial Sequence

<220>
<223>  DNMT1 Reverse Primer

<400>  8
gttttctaga cgtccattca c                                                  21


<210>  9
<211>  18
<212>  DNA
<213>  Artificial Sequence

<220>
<223>  TERT1 Forward Primer

<400>  9
cggaagagtg tctggagc                                                      18


<210>  10
<211>  19
<212>  DNA

<213>  Artificial Sequence

<220>
<223>  TERT1 Reverse Primer

<400>  10
ggatgaagcg gagtctgga                                                    19


<210>  11
<211>  24
<212>  DNA
<213>  Artificial Sequence

<220>
<223>  IL-6 Forward Primer

<400>  11
gatgagtaca aaagtcctga tcca                                              24


<210>  12
<211>  19
<212>  DNA
<213>  Artificial Sequence

<220>
<223>  IL-6 Reverse Primer

<400>  12
ctgcagccac tggttctgt                                                    19


<210>  13
<211>  24
<212>  DNA
<213>  Artificial Sequence

<220>
<223>  IDO Forward Primer

<400>  13
gcatttttca gtgttcttcg cata                                              24


<210>  14
<211>  24
<212>  DNA
<213>  Artificial Sequence

<220>
<223>  IDO Reverse Primer

<400>  14
tcatacacca gaccgtctga tagc                                              24


<210>  15
<211>  26
<212>  DNA
<213>  Artificial Sequence

<220>

<223>  TSG-6 Forward Primer

<400>  15
cccattgtga agccagggcc caactg                                          26


<210>  16
<211>  27
<212>  DNA
<213>  Artificial Sequence

<220>
<223>  TSG-6 Reverse Primer

<400>  16
ggaagctcat ctccacagta tcttccc                                         27


<210>  17
<211>  20
<212>  DNA
<213>  Artificial Sequence

<220>
<223>  P16INK4a Forward Primer

<400>  17
agcatggagc cttcggctga                                                 20


<210>  18
<211>  21
<212>  DNA
<213>  Artificial Sequence

<220>
<223>  P16ink4a Reverse Primer

<400>  18
ccatcatcat gacctggatc g                                               21


<210>  19
<211>  21
<212>  DNA
<213>  Artificial Sequence

<220>
<223>  RB Forward Primer

<400>  19
gctagcctat ctccggctaa a                                               21


<210>  20
<211>  20
<212>  DNA
<213>  Artificial Sequence

<220>
<223>  RB Reverse primer

<400>  20

ctggaaaagg gtccagatga                                                    20

<210>  21
<211>  20
<212>  DNA
<213>  Artificial Sequence

<220>
<223>  P21 Forward Primer

<400>  21
gagactctca gggtcgaaaa                                                    20

<210>  22
<211>  20
<212>  DNA
<213>  Artificial Sequence

<220>
<223>  P21 Reverse Primer

<400>  22
ttagggcttc ctcttggaga                                                    20

<210>  23
<211>  21
<212>  DNA
<213>  Artificial Sequence

<220>
<223>  TP53 Forward Primer

<400>  23
tgactgtacc accatccact a                                                  21

<210>  24
<211>  18
<212>  DNA
<213>  Artificial Sequence

<220>
<223>  TP53 Reverse Primer

<400>  24
aaacacgcac ctcaaagc                                                      18

<210>  25
<211>  20
<212>  DNA
<213>  Artificial Sequence

<220>
<223>  aSMA Forward Primer

<400>  25
gcagcccagc caagcactgt                                                    20

<210>  26
<211>  20
<212>  DNA
<213>  Artificial Sequence

<220>
<223>  aSMA Reverse Primer

<400>  26
tgggagcatc gtccccagca                                                    20


<210>  27
<211>  20
<212>  DNA
<213>  Artificial Sequence

<220>
<223>  RNA18S Forward Primer

<400>  27
atcggggatt gcaattattc                                                    20


<210>  28
<211>  20
<212>  DNA
<213>  Artificial Sequence

<220>
<223>  RNA18S Reverse Primer

<400>  28
ctcactaaac catccaatcg                                                    20


<210>  29
<211>  20
<212>  DNA
<213>  Artificial Sequence

<220>
<223>  P14ARF Forward Primer

<400>  29
tactgaggag ccagcgtcta                                                    20


<210>  30
<211>  20
<212>  DNA
<213>  Artificial Sequence

<220>
<223>  P14ARF Reverse Primer

<400>  30
tgcacgggtc gggtgagagt                                                    20


<210>  31
<211>  23
<212>  DNA

<213>  Artificial Sequence

<220>
<223>  CD47 Forward Primer

<400>  31
agaaggtgaa acgatcatcg agc                                              23


<210>  32
<211>  21
<212>  DNA
<213>  Artificial Sequence

<220>
<223>  CD47 Reverse Primer

<400>  32
ctcatccata ccaccggatc t                                                21


<210>  33
<211>  24
<212>  DNA
<213>  Artificial Sequence

<220>
<223>  Fpr Forward Primer

<400>  33
tctaccatct ccagagttct gtgg                                             24


<210>  34
<211>  24
<212>  DNA
<213>  Artificial Sequence

<220>
<223>  Fpr Reverse Primer

<400>  34
ttacatctac cacaatgtga acta                                             24

## Description

### Technical Field

The present invention relates to a method for producing mesenchymal stem cells, a marker for evaluating a therapeutic effect of mesenchymal stem cells, a method for determining a therapeutic effect of mesenchymal stem cells, a method for determining suitability of mesenchymal stem cells to be treated with a treatment for enhancing a therapeutic effect, and a cell preparation including mesenchymal stem cells.

### Background Art

Mesenchymal stem cells (hereinafter also referred to as MSCs), which are stem cells having multipotency and self-replication potency, can differentiate into not only cells belonging to the mesenchyme, such as osteoblasts, chondrocytes, adipocytes, and myocytes, but also neurocytes, hepatocytes, or the like, beyond the germ layer. Further, MSCs are known to have a paracrine effect and a cellular adhesive interaction by self-produced humoral factors. It is considered that, on the basis of these effects, MSCs exert a capability of repairing and regenerating target tissues and cells as well as a capability of controlling an immune response, for example, in anti-inflammation, thereby providing a therapeutic effect on various diseases.

MSCs are easily isolated and cultured and have a vigorous proliferation potential, thus the number of cells usable in transplantation can be achieved in a short period. Further, MSCs can be transplanted autologously without causing immunological rejection, MSCs cause few ethical problems, and allogeneic transplantation of MSCs is practical as there is no requirement for a pretreatment due to their low immunogenicity. For such reasons, MSCs are ideal materials for a cell transplantation therapy, and therapeutic applications of MSCs to a variety of diseases are under investigation.

In the process of establishing a cell transplantation therapy using MSCs, the present inventors have found that a patient, for example, a patient with diabetes, has abnormal MSCs, specifically, such a patient has MSCs in which the above diverse capabilities are lost, or these capabilities are reduced compared with those of normal MSCs, and thus their therapeutic effect on a disease is lost, or is reduced compared with that of the normal MSCs. Further, the present inventors have found that an extract from a mammalian fetal appendage can activate the abnormal MSCs and restore their therapeutic effect, and the MSCs activated in this manner can be used for an autologous transplantation therapy. On the basis of these findings, the present inventors have discovered an activator for the abnormal MSCs, which includes an extract from a mammalian fetal appendage as an active ingredient, and filed a patent application (Patent Literature 1). This activator is particularly significant as it enables autologous transplantation of MSCs even for a patient in a stage requiring a medical treatment.

The activator for the abnormal MSCs described in Patent Literature 1 can be produced as an extract from a mammalian fetal appendage, for example, a human umbilical cord tissue and placental tissue. Fortunately, a fetal appendage derived from a mammal including human is a relatively readily available biological sample and can be expected to have a certain quantity of supply. However, mammalian fetal appendages and activators extracted therefrom would be supplied hardly enough for making autologous transplantation of MSCs available to patients to whom cell transplantation therapies using MSCs are desirably applied, for example, to a large number of diabetes patients steadily increasing in the world.

### Citation List

### Patent Literatures

Patent Literature 1: WO2015/137419

### Summary of Invention

### Technical Problem

Studies by the present inventors have revealed, for example, that the activator can enhance a therapeutic effect of not only the abnormal MSCs but also MSCs derived from healthy persons, while some MSCs exist in which a therapeutic effect is hardly enhanced by the treatment with the activator. As described above, the activator including an extract from a mammalian fetal appendage as an active ingredient is a precious substance needed to be efficiently used, and it is not desirable that the activator is used for the MSCs in which a therapeutic effect is hardly enhanced even if it occurs just as a consequence. Further, the MSCs in which a therapeutic effect is not enhanced by the treatment with the activator cannot be expected to exert an effect on a patient to whom the MSCs are administered, thus it is desirable to confirm activity of MSCs prior to administration.

Thus, an object of the present invention is to provide a means for activating MSCs more efficiently in a production of the MSCs activated by using an activator including an extract from a mammalian fetal appendage as an active ingredient. Further, another object of the present invention is to provide a method for evaluating a therapeutic effect of MSCs, a method for determining whether MSCs separated from a subject have suitability to be treated with a treatment intended for enhancing a therapeutic effect of MSCs, such as a treatment with the activator, and a cell preparation including MSCs having a high therapeutic effect.

### Solution to Problem

The present inventors have found that, activated MSCs having a higher therapeutic effect can be produced with a smaller amount of an activator by activating the abnormal MSCs on a cell culture carrier having a three-dimensional structure formed of a fiber, and that even normal MSCs is activated by culturing them in the presence of the activator on the cell culture carrier. Further, the present inventors have found a specific gene expression change in MSCs having a high therapeutic effect by analyzing the gene expression profile thereof. Each of the following inventions has been completed on the basis of these findings.
(1) A method for producing activated mesenchymal stem cells, comprising an activation step of treating mesenchymal stem cells by an activator that includes an extract from a mammalian fetal appendage as an active ingredient, wherein, in the activation step, the mesenchymal stem cells are cultured in a medium containing the activator using a cell culture carrier having a three-dimensional structure formed of a fiber.
(2) The method according to (1), wherein the cell culture carrier has openings formed of the fiber having an average fiber diameter of from nanometer order to micrometer order on the cell contacting surface.
(3) The method according to (2), wherein an average diameter of the openings is from 500 nm to 1000 µm.
(4) The method according to any one of (1) to (3), wherein the cell culture carrier is formed by including a nanofiber made of a biodegradable polymer.
(5) The method according to any one of (1) to (4), wherein a concentration of the activator in the medium is from 1/10 to 1/100000 of a concentration of the activator in the medium in the activation step of culturing mesenchymal stem cells using a cell culture carrier having no three-dimensional structure formed of the fiber.
(6) The method according to any one of (1) to (5), wherein the concentration of the activator in the medium is from 0.01 µg/mL to 500 µg/mL in terms of protein.
(7) The method according to any one of (1) to (6), wherein the cells are passaged 2 times or less in the activation step.
(8) The method according to any one of (1) to (7), wherein the mesenchymal stem cells are derived from bone marrow.
(9) The method according to any one of (1) to (8), wherein the mesenchymal stem cells are separated from a subject with a disease.
(10) A cell preparation including mesenchymal stem cells having a CD47 positive cell proportion of 2% or less.
(11) The cell preparation according to (10), wherein the mesenchymal stem cells are the activated mesenchymal stem cells produced by the method according to any one of (1) to (9) .
(12) A method for producing a cell preparation including mesenchymal stem cells, including a step of concentrating CD47 negative mesenchymal stem cells from a mesenchymal stem cell population.
(13) A marker for evaluating a therapeutic effect of mesenchymal stem cells, the marker being selected from the group consisting of p16^{ink4a} and p14^{ARF} and having a positive correlation with the therapeutic effect of the mesenchymal stem cells.
(14) A marker for evaluating a therapeutic effect of mesenchymal stem cells, the marker being selected from the group consisting of CDK4, CDK6, RB, and CD47 and having a negative correlation with the therapeutic effect of the mesenchymal stem cells.
(15) A method for determining a therapeutic effect of mesenchymal stem cells, including: a measurement step of measuring a gene or protein expression level of at least one selected from the group consisting of p16^{ink4a}, p14^{ARF}, CDK4, CDK6, RB, and CD47 in test mesenchymal stem cells; a comparison step of comparing the measured gene or protein expression level with a gene or protein expression level in control mesenchymal stem cells; and a determination step of determining the test mesenchymal stem cells to have a higher therapeutic effect than the control mesenchymal stem cells if the gene or protein expression level of at least one selected from the group consisting of p16^{ink4a} and p14^{ARF} is higher in the test mesenchymal stem cells than in the control mesenchymal stem cells and/or if the gene or protein expression level of at least one selected from the group consisting of CDK4, CDK6, RB, and CD47 is lower in the test mesenchymal stem cells than in the control mesenchymal stem cells.
(16) The method according to (15), wherein
   the measurement step further includes measurement, in the test mesenchymal stem cells, of a gene or protein expression level of:
   (A) at least one selected from the group consisting of DNMT1, Nanog, SOX2, OCT4, IDO, TSG6, IL-6, and TERT; and/or
   (B) at least one selected from the group consisting of p53 and α-SMA, and
   the determination step determines that test mesenchymal stem cells have a higher therapeutic effect than the control mesenchymal stem cells if the gene or protein expression level of (A) is higher in the test mesenchymal stem cells than in the control mesenchymal stem cells and/or if the gene or protein expression level of (B) is lower in the test mesenchymal stem cells than in the control mesenchymal stem cells, in addition to the higher gene or protein expression level of at least one selected from the group consisting of p16^{ink4a} and p14^{ARF} in the test mesenchymal stem cells than in the control mesenchymal stem cells and/or the lower gene or protein expression level of at least one selected from the group consisting of CDK4, CDK6, RB, and CD47 in the test mesenchymal stem cells than in the control mesenchymal stem cells.
(17) The method according to (15) or (16), wherein, in the measurement step, a proportion of CD47 positive cells in the mesenchymal stem cells is measured instead of the gene or protein expression level of CD47, and, in the determination step, the test mesenchymal stem cells are determined to have the higher therapeutic effect than the control mesenchymal stem cells if the proportion of CD47 positive cells is lower in the test mesenchymal stem cells than in the control mesenchymal stem cells instead of the lower gene or protein expression level of CD47 in the test mesenchymal stem cells than in the control mesenchymal stem cells.
(18) The method according to any one of (15) to (17), wherein the test mesenchymal stem cells are mesenchymal stem cells cultured in a medium including an activator containing an extract from a mammalian fetal appendage as an active ingredient.
(19) The method according to any one of (15) to (18), wherein the test mesenchymal stem cells are mesenchymal stem cells cultured using a cell culture carrier having a three-dimensional structure formed of a fiber.
(20) The method according to any one of (15) to (19), wherein the test mesenchymal stem cells are mesenchymal stem cells separated from a subject being to undergo an autologous transplantation therapy of the mesenchymal stem cells.
(21) The method according to any one of (15) to (20), wherein the test mesenchymal stem cells are derived from bone marrow.
(22) A method for determining suitability of mesenchymal stem cells to be treated with a treatment for enhancing a therapeutic effect of mesenchymal stem cells, including: a measurement step of measuring a gene or protein expression level of at least one selected from the group consisting of p16^{ink4a}, p14^{ARF}, CDK4, CDK6, RB, and CD47 in treated mesenchymal stem cells; a comparison step of comparing the measured gene or protein expression level with a gene or protein expression level in untreated mesenchymal stem cells; and a determination step of determining the mesenchymal stem cells to be suitable to be treated with the treatment if the gene or protein expression level of at least one selected from the group consisting of p16^{ink4a} and p14^{ARF} is higher in the treated mesenchymal stem cells than in the untreated mesenchymal stem cells and/or if the gene or protein expression level of at least one selected from the group consisting of CDK4, CDK6, RB, and CD47 is lower in the treated mesenchymal stem cells than in the untreated mesenchymal stem cells.
(23) The method according to (22), wherein
   the measurement step further includes measurement, in each of the treated mesenchymal stem cells and the untreated mesenchymal stem cells, of a gene or protein expression level of:
   (A) at least one selected from the group consisting of DNMT1, Nanog, SOX2, OCT4, IDO, TSG6, IL-6, and TERT; and/or
   (B) at least one selected from the group consisting of p53 and α-SMA, and
   the determination step determines that the mesenchymal stem cells are suitable to be treated with the treatment if the gene or protein expression level of (A) is higher in the treated mesenchymal stem cells than in the untreated mesenchymal stem cells and/or if the gene or protein expression level of (B) is lower in the treated mesenchymal stem cells than in the untreated mesenchymal stem cells, in addition to the higher gene or protein expression level of at least one selected from the group consisting of p16^{ink4a} and p14^{ARF} in the treated mesenchymal stem cells than in the untreated mesenchymal stem cells and/or the lower gene or protein expression level of at least one selected from the group consisting of CDK4, CDK6, RB, and CD47 in the treated mesenchymal stem cells than in the untreated mesenchymal stem cells.
(24) The method according to (22) or (23), wherein, in the measurement step, a proportion of CD47 positive cells in the mesenchymal stem cells is measured instead of the gene or protein expression level of CD47, and, in the determination step, the mesenchymal stem cells are determined to be suitable to be treated with the treatment if the proportion of CD47 positive cells is lower in the treated mesenchymal stem cells than in the untreated mesenchymal stem cells instead of the lower gene or protein expression level of CD47 in the treated mesenchymal stem cells than in the untreated mesenchymal stem cells.
(25) The method according to any one of (22) to (24), wherein the treatment is culturing the mesenchymal stem cells in a medium including an activator containing an extract from a mammalian fetal appendage as an active ingredient.
(26) The method according to any one of (22) to (25), wherein the treatment is culturing the mesenchymal stem cells using a cell culture carrier having a three-dimensional structure formed of a fiber.
(27) The method according to any one of (22) to (26), wherein the mesenchymal stem cells are mesenchymal stem cell separated from a subject being to undergo an autologous transplantation therapy of the mesenchymal stem cells.
(28) The method according to any one of (22) to (27), wherein the mesenchymal stem cells are derived from bone marrow.

### Advantageous Effects of Invention

According to the method for producing MSCs of the present invention, MSCs can be activated by using an activator in an amount much less than conventionally used, making it possible to save the consumption of the precious activator. Further, the cell preparation of the present invention which can be produced by the production method has a high therapeutic effect, making it possible to reduce the number of cells required for therapy or the like. Reducing the number of cells to be administered leads to a reduction in the number of passage times in cell culture, causing an effect such as shortening the period needed for producing the cell preparation and cutting costs. Further, according to the therapeutic effect marker and determination method of the present invention, a therapeutic effect of MSCs can be determined prior to administration to a subject. In addition, according to the suitability determination method of the present invention, suitability of MSCs to be treated with a treatment such as an activation treatment can be determined prior to preparation of MSCs in an amount required for therapy.

### Brief Description of Drawings

Fig. 1 includes a diagram illustrating a result of a cluster analysis of gene expression profiles of MSCs derived from a patient with osteoarthritis of hip (hereinafter referred to as "OA-MSCs") cultured in the presence of an activator on cell culture carriers having a three-dimensional structure formed of a fiber (hereinafter also referred to as "three-dimensional culture carriers").
Fig. 2 includes a graph showing relative expression levels of marker genes in OA-MSCs cultured in the presence of the activator on a cell culture carrier having a conventional two-dimensional structure (hereinafter referred to as "two-dimensional culture carrier") and OA-MSCs cultured in the presence of the activator on the three-dimensional culture carrier, in comparison with those of the marker genes in OA-MSCs cultured in the absence of the activator on the two-dimensional culture carrier.

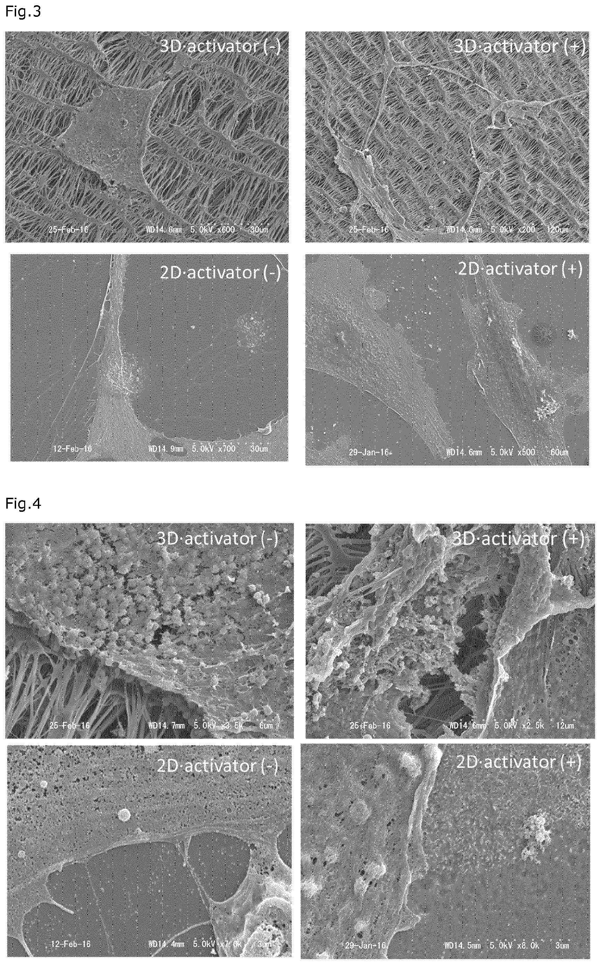

Fig. 3 includes electron microscope observation photographs showing appearance of the OA-MSCs cultured on the three-dimensional culture carrier and the OA-MSCs cultured on the two-dimensional culture carrier. The photograph on the upper left corresponds to the OA-MSC cultured on the three-dimensional culture carrier without the activator (600X magnification), the photograph on the upper right corresponds to the OA-MSC cultured on the three-dimensional culture carrier with the activator (200X magnification), the photograph on the lower left corresponds to the OA-MSC cultured on the two-dimensional culture carrier without the activator (700X magnification), and the photograph on the lower right corresponds to the OA-MSC cultured on the two-dimensional culture carrier with the activator (500X magnification).
Fig. 4 includes strongly enlarged electron microscope observation photographs showing appearance of the OA-MSCs cultured on the three-dimensional culture carrier and the OA-MSC cultured on the two-dimensional culture carrier. The photograph on the upper left corresponds to the OA-MSC cultured on the three-dimensional culture carrier without the activator (3500X magnification), the photograph on the upper right corresponds to the OA-MSC cultured on the three-dimensional culture carrier with the activator (2500X magnification), the photograph on the lower left corresponds to the OA-MSC cultured on the two-dimensional culture carrier without the activator (7000X magnification), and the photograph on the lower right corresponds to the OA-MSC cultured on the two-dimensional culture carrier with the activator (8000X magnification).
Fig. 5 includes a diagram illustrating a result of a cluster analysis of gene expression profiles of OA-MSCs cultured in the presence of the activator on a cell culture carrier of Comparative example or another three-dimensional culture carrier in combination with the gene expression profiles on the three-dimensional culture carriers.
Fig. 6 includes an electron microscope observation photograph (3500X magnification) showing appearance of the OA-MSC cultured with the activator on another three-dimensional culture carrier.
Fig. 7 includes a graph showing relative expression levels of OCT4, SOX2, and TSG-6 genes in MSCs derived from a healthy person cultured in the presence of the activator on the two-dimensional culture carrier and MSCs derived from the healthy person cultured in the presence of the activator on the three-dimensional culture carrier, in comparison with the expression levels of those genes in MSCs derived from the healthy person cultured in the absence of the activator on the two-dimensional culture carrier.
Fig. 8 includes a graph showing remaining defect areas in skin defect model rats on Day 3 and Day 7 after skin excision, to which rats OA-MSCs treated with the activator were administered.
Fig. 9 includes photographs showing skin defect parts in the skin defect model rats on Day 7 after skin excision, to which rats OA-MSCs treated with the activator were administered.
Fig. 10 includes a graph showing a result of measuring a rupture stress of skin tissues in the skin defect model rats on Day 21 after skin excision, to which rats OA-MSCs treated with the activator were administered.
Fig. 11 includes a graph showing a result of a learning test with Morris water maze for Alzheimer's disease model mice to which OA-MSCs treated with the activator were administered. Escape latency on the vertical axis indicates a time for the mice to reach a platform placed in a pool, while the horizontal axis indicates the number of days from the start of the learning test.
Fig. 12 includes a graph showing a result of a memory test with Morris water maze for Alzheimer's disease model mice to which OA-MSCs treated with the activator were administered. Time in quadrant on the vertical axis indicates a proportion of a time swimming in the quadrant region where the platform existed in the learning process to the total swimming time.
Fig. 13 includes a graph showing a change in serum creatinine in rats with diabetic nephropathy after treated with an anticancer drug, to which rats OA-MSCs treated with the activator were transplanted.
Fig. 14 includes a graph showing a change in survival rates, of the rats with diabetic nephropathy after treated with an anticancer drug, up to 11 weeks after transplantation, to which rats OA-MSCs treated with the activator were transplanted. The thick lines indicate an average value of each group and the thin lines indicate a 95% confidence interval (CI) of each group.
Fig. 15 includes a diagram illustrating a result of a cluster analysis of gene expression profiles of OA-MSCs (n = 10).
Fig. 16 includes a diagram illustrating a result of a cluster analysis of gene expression profiles of OA-MSCs (n = 10).
Fig. 17 includes diagrams illustrating a correlation between a relative expression level of each of OCT4, SOX2, NANOG, IDO, and TSG6 and a relative expression level of p16^{ink4a}. The relative expression levels of these genes were calculated by comparing the gene expression levels in OA-MSCs treated with the activator with those in untreated MSCs.
Fig. 18 includes a diagram illustrating a correlation between a relative expression level of p14^{ARF} and a relative expression level of p16^{ink4a}. The relative expression levels of the gene were calculated by comparing the gene expression levels in OA-MSCs treated with the activator with those in the untreated MSCs.
Fig. 19 includes graphs showing relative expression levels of OCT4, p16^{ink4a}, and RB genes in OA-MSCs (BM-021, BM-022, and BM-023). In the figure, OA-MSCs treated with the activator are represented by PE+ or PE+ MSC, while the untreated OA-MSCs are represented by PE- or PE- MSC (the same applies to the following drawings).
Fig. 20 includes a graph showing relative expression levels of OCT4, SOX2, and p16^{ink4a} genes in OA-MSCs (BM-010) treated with the activator on the three-dimensional culture carrier.
Fig. 21 includes a graph showing a relative expression level of CD47 gene in MSCs derived from a patient with rheumatoid arthritis (RA), MSCs derived from an aged person (Aged), MSCs derived from a patient with liver cirrhosis, and MSCs derived from a young person (Young), all of which were treated with the activator on the three-dimensional culture carrier. The relative exdpression levels are represented in comparison with the expression level of CD47 gene in MSCs cultured in the absence of the activator on the two-dimensional culture carrier.
Fig. 22 includes histograms showing an expression level of CD47 on the cellular surface of untreated MSCs derived from a young person and untreated MSCs derived from an aged person, respectively.
Fig. 23 includes histograms showing an expression level of CD47 on the cellular surface of MSCs derived from a young person and MSCs derived from an aged person, respectively, both of which MSCs were treated with the activator on the three-dimensional culture carrier.
Fig. 24 includes a graph showing a relative expression level of p16^{ink4a} gene in OA-MSCs (BM-008, BM-021, and BM-005).
Fig. 25 includes a graph showing phagocytic ability of RAW264.7 cells co-cultured with OA-MSCs (BM-008, BM-021, and BM-005).
Fig. 26 includes a graph showing a relative expression level of Fpr2 (Formyl Peptide Receptor-2) gene in RAW264.7 cells co-cultured with OA-MSCs (BM-008, BM-021, and BM-005).
Fig. 27 includes a graph showing relative expression levels of OCT4, SOX2, and p16^{ink4a} genes in OA-MSCs (BM-021).
Fig. 28 includes graphs showing proportions of total macrophages (MΦ), M1 macrophages, and M2 macrophages to the total number of mononuclear cells excluding dead cells in polymyositis model mice. OA-MSCs (BM-021) treated with the activator or untreated, or PBS was locally administered to the mice.
Fig. 29 includes graphs showing a hind leg muscle function in polymyositis model mice. OA-MSCs (BM-021) treated with the activator or untreated was locally administered to the mice.
Fig. 30 includes a graph showing relative expression levels of OCT4, SOX2, and p16^{ink4a} genes in OA-MSCs (BM-008) .
Fig. 31 includes a graph showing a muscle function in polymyositis model mice. OA-MSCs (BM-008) treated with the activator or untreated, or PBS was systemically administered to the mice.
Fig. 32 includes HE staining images of muscle tissues in polymyositis model mice. OA-MSCs (BM-008) treated with the activator or untreated, or PBS was systemically administered to the mice.
Fig. 33 includes a diagram illustrating muscle cross-sectional areas in polymyositis model mice. OA-MSCs (BM-008) treated with the activator or untreated, or PBS was systemically administered to the mice.
Fig. 34 includes a graph showing relative expression levels of OCT4, SOX2, and p16^{ink4a} genes in OA-MSCs (BM-021) treated with the activator on the two-dimensional culture carrier or the three-dimensional culture carrier.
Fig. 35 includes a graph showing relative expression levels of OCT4, SOX2, NANOG, p16^{ink4a}, p14^{ARF}, IDO, α-SMA, and TERT genes in OA-MSCs treated with the activator, which were transplanted to the rats with diabetic nephropathy after treated with an anticancer drug. The relative expression levels are represented in comparison with the expression levels of these genes in MSCs cultured in the absence of the activator on the two-dimensional culture carrier.
Fig. 36 includes images showing expression of p16^{ink4a} in CDKN2A knockout OA-MSCs treated with the activator on the three-dimensional culture carrier.
Fig. 37 includes laminin staining images (left) and a diagram illustrating muscle cross-sectional areas (right) of muscle tissues in polymyositis model mice. CDKN2A knockout OA-MSCs treated with the activator on the three-dimensional culture carrier were locally administered to the triceps surae muscle in the mice.
Fig. 38 includes a graph showing a change in wound areas from Day 3 to Day 9 after skin excision (left) and a representative photograph of skin defect parts on Day 9 after skin excision (right) in the skin defect model mice to which OA-MSCs over-expressing CD47 were administered.
Fig. 39 includes a graph showing a result of learning tests with Morris water maze for Alzheimer's disease model mice to which either of two types of OA-MSCs having different CD47 positive cell proportions were administered.
Fig. 40 includes a graph showing a result of a memory test with Morris water maze for Alzheimer's disease model mice to which either of two types of OA-MSCs having different CD47 positive cell proportions were administered.
Fig. 41 includes a graph showing relative expression levels of OCT4, SOX2, and TSG-6 genes in OA-MSCs cultured on the two-dimensional culture carrier or the three-dimensional culture carrier in the presence of another activator obtained by a different extraction method, and in OA-MSCs cultured on the three-dimensional culture carrier in the absence of the activator. The relative expression levels are represented in comparison with the expression levels of these genes in MSCs derived from a healthy person cultured in the absence of the activator on the two-dimensional culture carrier.
Fig. 42 includes a graph showing relative expression levels of p16^{ink4a} gene in OA-MSCs cultured on the two-dimensional culture carrier or the three-dimensional culture carrier in the presence of another activator obtained by a different extraction method, and in OA-MSCs cultured on the three-dimensional culture carrier in the absence of the activator. The relative expression levels are represented in comparison with the expression levels of these genes in MSCs derived from a healthy person cultured in the absence of the activator on the two-dimensional culture carrier.

### Description of Embodiments

### Method for Producing Activated MSCs

A first aspect of the present invention relates to a method for producing activated MSCs including an activation step of treating MSCs by an activator that includes an extract from a mammalian fetal appendage as an active ingredient, in which, in the activation step, the MSCs are cultured in a medium containing the activator using a cell culture carrier having a three-dimensional structure formed of a fiber.

MSCs used in the present invention may be separated from a healthy subject or a subject with a disease. Further, MSCs used in the present invention may be obtained by inducing differentiation from pluripotent stem cells such as induced pluripotent stem cells (iPS cells), embryonic stem cells (ES cells), embryonal carcinoma cells (EC cells), and embryonic germ stem cells (EG cells).

The terms "subject", "MSCs separated from a subject with a disease", and "extract from a mammalian fetal appendage" in the present specification are all to be construed as having the same meanings as those of respective terms described in WO 2015/137419 pamphlet as Patent Literature 1 and the U.S. Patent Application Publication No. 2017/0071984 for the corresponding US application. These documents are incorporated herein in their entireties by reference. Nevertheless, an outline of a meaning of each term in these documents and the present specification is described below.

The term "subject" means any animal having MSCs. The subject is preferably an individual of a mammal, for example, an individual of a primate such as human and a chimpanzee, a rodent such as a mouse, a rat, a guinea pig, and a hamster, the Artiodactyla such as a cow, a goat, a sheep, and a pig, the Perissodactyla such as a horse, as well as a rabbit, a dog, and a cat, and is more preferably an individual of human.

As described in Patent Literature 1, it is known that MSCs derived from a subject with a certain disease and an aged subject have a lower therapeutic effect than MSCs derived from a healthy person, thus a high therapeutic effect cannot be expected by simply performing autologous transplantation of the MSCs for these subjects. On the other hand, according to a production method of the first aspect, it becomes possible to activate such MSCs having a low therapeutic effect and enhance their therapeutic effect. A disease with which a subject having MSCs with a low therapeutic effect is affected is a chronic disease, and examples of such a chronic disease are described in Patent Literature 1 as diseases that cause abnormal MSCs.

Considering the safety in the subsequent cell transplantation therapy, MSCs are preferably collected from an individual of the same or relative species of an individual to which the MSCs are to be administered. For example, in a case where MSCs are transplanted into a human individual, MSCs collected from human as the same species are preferably used, and MSCs collected from the same human individual to which the MSCs to be administered, that is, autologous MSCs, are more preferably used.

MSCs used in the present invention can be collected by a general method from a sample, such as a bone marrow fluid, an adipose tissue, a tissue from the fetal appendage, and dental pulp, from a subject. For example, when using a bone marrow fluid as a sample, MSCs can be separated by a known method, such as density gradient centrifugation and bone marrow seeding. In one preferred embodiment of the present invention, MSCs are derived from bone marrow.

The term "extract from a mammalian fetal appendage" refers to an extract prepared, for example, by immersing a fetal appendage of a mammal, preferably human, as it is or after cutting or crushing, in an extraction medium such as distilled water, a physiological saline solution, a phosphate-buffered physiological saline solution, and a medium commonly used for cell culture. A fetal appendage is expelled from a mother body as an afterbirth after expulsion of a fetus, or taken out from a mother body by cesarean section, and is preferably an umbilical cord tissue, a placental tissue, or a placental membrane. It is particularly preferable that the extract is free from a cell having proliferation potency derived from the donar mammal. As specific extraction operations and conditions, operations and conditions described in Patent Literature 1 may be used.

Further, the "extract from a mammalian fetal appendage" can also be prepared by subjecting a fetal appendage to a treatment normally used by those skill in the art in extracting a physiologically active substance from a fetal appendage, typically a placenta. Examples of such a treatment include hydrolysis using an acid or an enzyme. Examples of such an extract include a human placenta extract "Melsmon" commercially available from MELSMON Pharmaceutical Co., Ltd., a human placenta extract "Laennec" commercially available from JAPAN BIO PRODUCTS Co., Ltd., other commercially available placenta preparations, and various commercially available products labelled a placenta extract.

The term "activator including an extract from a mammalian fetal appendage as an active ingredient" in the present invention refers to an agent for enhancing a therapeutic effect of MSCs, which includes the extract described above as an active ingredient, and such an agent is hereinafter referred to as an "activator". The term "activation" herein means that MSCs subjected to a certain treatment gain a higher therapeutic effect compared with a case of not being treated.

In the production method of the first aspect, an activation step is performed by culturing MSCs in vitro in a medium containing the activator using a cell culture carrier having a three-dimensional structure formed of a fiber. Specifically, the activation step is performed by culturing MSCs in the presence of the activator using the three-dimensional culture carrier as a scaffold.

In a preferred embodiment, the three-dimensional culture carrier is formed of a fiber having an average fiber diameter of from nanometer (nm) order to micrometer (µm) order. The term "average fiber diameter" herein refers to an arithmetic average value of fiber diameters measured as a length in a direction orthogonal to the length direction of the fiber when the culture carrier is observed from the cell adhesion surface side, typically, from the upper side. In a more preferable embodiment, the three-dimensional culture carrier has openings formed of the fiber having an average fiber diameter of from nanometer (nm) order to micrometer (µm) order on the cell contacting surface. The term "opening" herein refers to a concavity present on the cell contacting surface of the carrier, formed of the fiber described above. Note that, unless otherwise specifically stated, the term "average" refers to a "number average" in the present specification.

In a case where the fibers are in contact with each other, an average diameter of the openings refers to an average value of diameters of figures having the fibers as outlines, recognized when the culture carrier was observed from the upper side. The diameter of the figure corresponds to an arithmetic average value of lengths of diagonal lines connecting each vertices in a case where the figure is a polygon, corresponds to a diameter in a case where the figure is a circle, and corresponds to a longer diameter in a case where the figure is an ellipse or has a shape similar thereto.

Further, in a case where the fibers are not in contact with each other, an average diameter of the openings refers to an average flow pore diameter obtained by a method defined by ASTM-F316. The average flow pore diameter can be measure by a mean flow point method using, for example, a porometer (manufactured by Coulter Electronics, etc.).

The average fiber diameter of the fibers constituting the three-dimensional culture carrier can be within a range of from nm order to µm order, preferably from 10 nm to 500 µm, more preferably from 10 nm to 300 µm. In a particular embodiment, the average fiber diameter can be within a range, for example, from 10 nm to 1 µm, from 100 nm to 1 µm, from 500 nm to 1 µm, from 1 µm to 10 µm, from 1 µm to 100 µm, from 1 µm to 300 µm, or from 1 µm to 500 µm, preferably from 10 nm to 1 µm, from 1 µm to 10 µm, or from 10 µm to 300 µm. In the present invention, a fiber generally called a nanofiber can also be used.

In the three-dimensional culture carrier having the openings on the contact surface with cells, the average diameter of the openings can be within a range of from 500 nm to 1000 µm, preferably from 700 nm to 600 µm, more preferably from 900 nm to 400 µm. In a particular embodiment, the average diameter of the openings can be within a range, for example, from 500 nm to 100 µm, from 5 µm to 100 µm, from 10 µm to 100 µm, from 20 µm to 100 µm, from 100 µm to 200 µm, from 100 µm to 400 µm, or from 100 µm to 600 µm, preferably from 500 nm to 100 µm or from 100 µm to 400 µm.

In a particular embodiment, the three-dimensional culture carrier has a void ratio of 60% or more, preferably 70% or more, more preferably 75% or more, particularly preferably 80% or more.

In another embodiment, an average area of the openings in the three-dimensional culture carrier is from 0.1 to 100 µm², preferably from 0.2 to 60 µm², more preferably from 0.5 to 30 µm². In a case where the opening is a pore, the opening area corresponds to a pore area.

The three-dimensional culture carrier has a structure in which the fibers are three-dimensionally integrated, that is, the fibers are stacked up in three-dimensional directions. The fibers may be arranged regularly or irregularly, and, further, the fibers may be connected to each other or may not be connected to each other.

The three-dimensional culture carrier is only required to have a three-dimensional structure formed of a fiber on its cell adhesion surface, and may include a member serving as a base in a portion not contacting with cells, typically, beneath the three-dimensional structure formed of a fiber. The base member may be any structure as long as it can support the three-dimensional structure. Examples of the base member can include nonwoven fabric, knit fabric, woven fabric, and a porous scaffold material.

The cell adhesion surface of the three-dimensional culture carrier is only required to be a portion the main part of which has a three-dimensional structure formed of fibers. Specifically, it is only required that, when the culture carrier is observed from the cell adhesion surface side, typically, from the upper side, 50% or more of the area of the culture carrier is occupied by a portion having the three-dimensional structure formed of a fiber. Thus, the three-dimensional culture carrier can include, in a part of the cell adhesion surface, a portion which does not have the "three-dimensional structure formed of a fiber", for example, a flat film-like portion or the like not having a three-dimensional structure, in a range in which the area of such a portion in the adhesion surface is less than 50%, preferably less than 40%, further preferably less than 30%.

A material forming the fiber is not particularly limited, and examples of the material include a polymer compound such as polyfluoroethylene and polystyrene, an inorganic compound such as silica, and a biodegradable polymer. The biodegradable polymer may be any biodegradable polymer that has biocompatibility and can maintain the three-dimensional structure on the culture carrier for a desired period. Examples of the biodegradable polymer include a synthetic polymer material such as polyglycolic acid, polylactic acid, polyethylene glycol, polycaprolactone, polydioxanone, and, further, a copolymer of the above, such as a lactic acid-glycolic acid copolymer; an inorganic material such as β-tricalcium phosphate and calcium carbonate; and a natural polymer material such as collagen, gelatin, alginic acid, hyaluronic acid, agarose, chitosan, fibrin, fibroin, chitin, cellulose, and silk.

A shape of the three-dimensional culture carrier is not limited as long as cells can be brought into contact with the three-dimensional structure formed of a fiber during culturing. The three-dimensional culture carrier may be formed as an insertion type to be placed in a cell culture container for use or formed in a shape in which the three-dimensional structure formed of a fiber is integrally molded with an inner surface of the cell culture container, for example, a bottom surface of the well or the like.

Examples of the three-dimensional culture carrier usable in the present invention include VECELL (registered trademark) available from Vessel Inc. (polytetrafluoroethylene, average fiber diameter: < 1 µm, average pore area: 1 to 20 µm², average diameter of openings: 20 to 100 µm, void ratio: 80 to 90%), Cellbed (registered trademark) available from Japan Vilene Company, Ltd. (high purity silica fiber, average fiber diameter: 1 µm, average flow pore diameter: 7 to 8 µm, void ratio: > 95%), 3D Insert-PS series available from 3D Biotek LLC. (polystyrene fiber, average fiber diameter: about 150 µm for PS-200, about 300 µm for PS-400, average diameter of openings: 200 µm for PS-200, 400 µm for PS-400), a cell culture base material described in WO 2014/196549 pamphlet, a cell culture base material described in WO 2016/068266 pamphlet and U.S. Patent Application Publication No. 2017/319747 for the corresponding US application (biodegradable polymer, average fiber diameter of from 50 nm to 5 µm), a cell culture base material described in Liu L, Kamei K et al., Biomaterials 124 (2017) 47-54 (polyglycolic acid, average fiber diameter: 345 ± 91 nm, average pore area: 0.68 ± 0.02 µm², average diameter of openings calculated from average pore area: 0.93 µm ((0.68 µm²/π)^{1/2}) × 2), and a commercially available tissue reinforcement material such as NEOVEIL and NEOVEIL NANO (GUNZE Ltd.). Each of the above references is incorporated herein its entirety by reference.

Cultivation of MSCs on the three-dimensional culture carrier is performed for from 24 hours to 144 hours, preferably from 24 hours to 96 hours, more preferably from 48 hours to 96 hours. A culture temperature and a gas concentration in the activation step only need to be within ranges of temperature and gas concentration normally used for culturing MSCs, and the temperature is, for example, from 25°C to 37°C, preferably from 30°C to 37°C, more preferably 37°C, and an oxygen concentration is, for example, from 2% to 30%, preferably from 2% to 20%. The activation treatment can be performed multiple times until activation is sufficiently achieved.

A concentration of the activator in the medium in the activation step only needs to be from 0.01 µg/mL to 500 µg/mL as a final concentration converted in terms of protein. It is preferably from 0.02 µg/mL to 300 µg/mL, 0.03 µg/mL to 200 µg/mL, more preferably from 0.04 µg/mL to 100 µg/mL, and in a particular embodiment, it can be from 0.05 µg/mL to 10 µg/mL. As is shown by comparison of the more preferable concentration of the activator, from 0.1 mg/mL (100 µg/mL) to 5 mg/mL (5000 µg/mL), exemplified in Patent Literature 1 with the concentration of the activator in the above particular embodiment, the concentration of the activator can be reduced to from 1/10 to 1/100000 of the conventionally used concentration by using the three-dimensional culture carrier. A lower limit of the concentration range of the activator is, for example, 1/100000, 1/50000, 1/20000, 1/10000, 1/5000, or 1/2000 of the concentration used in a case of using a conventional two-dimensional culture carrier, and an upper limit of the concentration range of the activator is, for example, 1/10, 1/20, 1/50, 1/100, 1/200, 1/500, or 1/1000 of the concentration used in a case of using a conventional two-dimensional culture carrier.

As the medium, a medium commonly used for culturing MSCs, for example, α-MEM, DMEM, or the like can be used. These media may contain various components required for the growth of MSCs, for example, a serum component or the like, as long as the activation is not prevented.

As shown in Example below, MSCs cultured in the presence of the activator on the three-dimensional culture carrier have a higher therapeutic effect as compared with MSCs cultured in the presence of the activator on a culture carrier not having a three-dimensional structure formed of a fiber, for example, a two-dimensional culture carrier made of polystyrene or the like such as a Corning (registered trademark) Costar (registered trademark) cell culture plate (Thermo Fisher Scientific). The MSCs having a higher therapeutic effect can be expected to exhibit the effect with a smaller number of cells, thus MSCs can be passaged less times in the activation step of the present invention than in an activation step of culturing the MSCs using a cell culture carrier not having a three-dimensional structure formed of a fiber. In the activation step of the present invention, MSCs are passaged preferably 2 times or less, further, MSCs may be passaged 1 time or even zero time. The passage number of the activated MSCs produced by the production method of the first aspect is preferably 3 or less, that is, the activated MSCs are P3 or less, further, the activated MSCs may be P2 or even P1.

Whether MSCs are activated or not can be determined in accordance with the method and standard described in Patent Literature 1. For example, in an evaluation system which reflects a disease, such as a disease model animal and a disease model animal-derived cell, if a therapeutic effect of MSCs cultured in the presence of the activator on the three-dimensional culture carrier (hereinafter also referred to as "3D activation-treated MSCs") is higher than that of MSCs cultured in the absence of the activator on a two-dimensional culture carrier (hereinafter also referred to as "control MSCs"), it is determined that the 3D activation-treated MSCs are activated.

Further, activation can be determined by using an evaluation indicator such as morphology of a cell and an intracellular organelle, cell proliferation potency, differentiation potency, and protein expression levels, gene expression levels, and extracellular secretion levels of various factors such as a growth factor, a differentiation-related factor, and cytokine/chemokine, which are known as a marker for MSCs having a therapeutic effect.

For example, if the cell proliferation potency or differentiation potency of the 3D activation-treated MSCs are higher than that of the control MSCs, it can be determined that the 3D activation-treated MSCs have a high therapeutic effect, that is, the 3D activation-treated MSCs are activated.

Further, it can also be determined that the 3D activation-treated MSCs have a high therapeutic effect, that is, are activated, if a gene or protein expression level of a factor involved in sternness (e.g., DNMT1, Nanog, SOX2, OCT4, etc.), a factor involved in immunoregulation/anti-inflammatory function (e.g., IDO, TSG6, IL-6, etc.), or a factor involved in a telomerase activity (e.g., TERT, etc.) is higher in the 3D activation-treated MSCs than in the control MSCs or if a gene or protein expression level of a factor involved in cellular senescence (e.g., P53, etc.) or a factor involved in a cytoskeleton (e.g., α-SMA, etc.) is lower in the 3D activation-treated MSCs than in the control MSCs.

Further, it can also be determined that the 3D activation-treated MSCs have a high therapeutic effect, that is, are activated, if, in the 3D activation-treated MSCs, an increase in a cell thickness, an increase in the number of projections, an enhancement in formation of network structures, an enhancement in formation of secretory vesicles, or the like are observed.

In addition, as described below, it can also be determined whether or not a therapeutic effect of the 3D activation-treated MSCs is higher than that of the control MSCs, that is, whether or not the 3D activation-treated MSCs are activated, using, as an indicator, gene or protein expression of p16^{ink4a} and p14^{ARF} having a high positive correlation with a therapeutic effect of MSCs, newly discovered by the present inventors, as well as gene or protein expression of CDK4, CDK6, RB, and CD47 having a high negative correlation with a therapeutic effect of MSCs, also newly discovered by the present inventors.

The activated MSCs may be maintained in an undifferentiated state or differentiated into desired cells. The MSCs can be maintained in an undifferentiated state by culturing the MSCs using a medium suitable for maintaining an undifferentiated state, for example, HyClone AdvanceSTEM Mesenchymal Stem Cell Expansion Kit (Thermo Fisher Scientific), MesenCult (trade mark) MSC Basal Medium (STEMCELL Technologies Inc.), Stromal Cellutions (trade mark) Media (DV Biologics LLC), a medium kit for MSC (MSCGM BulletKit, Lonza), or the like. Further, the MSCs can be differentiated by a generally known method such as a cultivation in a differentiation-inducing medium to which a factor having a differentiation-inducing effect into desired cells is added. For example, as a differentiation-inducing factor, Bone Morphogenetic Proteins (BMP) 4, BMP2 or the like is used for differentiation into osteoblasts, and dexamethasone, 3-isobutyl-1-methyl xanthine, insulin or the like is used for differentiation into adipocytes.

Further, the MSCs can be stored by a general procedure such as cryopreservation before and after a treatment such as activation, proliferating cultivation and differentiation-inducing cultivation. For example, it is possible that the activated MSCs are cultured to proliferate, the resultant cells are aliquoted in portions in a constant number and stored, and then a required amount of cells is thawed and used in each administration.

### Therapeutic Effect Marker of MSCs and Method for Determining Therapeutic Effect of MSCs

Another aspect of the present invention relates to a marker for evaluating a therapeutic effect of MSCs, which is selected from the group consisting of p16^{ink4a} and p14^{ARF} and has a positive correlation with the therapeutic effect of the MSCs. Further, still another aspect of the present invention relates to a marker for evaluating a therapeutic effect of MSCs, which is selected from the group consisting of CDK4, CDK6, RB, and CD47 and has a negative correlation with the therapeutic effect of the MSCs.

p16^{ink4a} is a cyclin-dependent kinase inhibitor having ankyrin repeats. p16^{ink4a} is known as one of cancer inhibitory factors and is also known as a senescence-related gene of which an expression level is increased when a cell reaches the end of lifetime division span or DNA is damaged by exposure to a carcinogenic stress or the like (E. Hara et al., Mol. Cell. Biol., 1996, Vol. 16, p. 859-867; B. G. Childs et al., Nature Medicine, 2015, vol. 21, p. 1424-1435).

p16^{ink4a} constitutes a signal transduction pathway called a p16^{ink4a}-RB pathway together with cyclin-dependent kinases CDK4 and CDK6 and a cell cycle-regulating factor RB (retinoblastoma protein, a gene encoding RB is also referred to as Rb). In this pathway, p16^{ink4a} specifically binds to CDK4/6 to inhibit their functions. Inhibition of CDK4/6 increases a level of dephosphorylated RB as an active form and thereby prevents progression of cell cycle. Hereinafter, these factors are collectively referred to as p16^{ink4a}-RB pathway factors.

Base sequences of human p16^{ink4a} gene and p16^{ink4a} mRNA translated to the protein are registered in a database named GenBank/NCBI under accession numbers NM_000077 and NM_001195132, respectively. An amino acid sequence of the protein is registered in a database named GenPept (NCBI Protein Database) under an accession number NP_000068.

Base sequences of human CDK4 and CDK6 genes and CDK4 and CDK6 mRNA translated to the protein are registered in a database named GenBank/NCBI under accession numbers NM_000075 and NM_001259, respectively. Amino acid sequences of the proteins are registered in a database named GenPept (NCBI Protein Database) under accession numbers NP_000066 and NP_001138778 (or NP_001250).

Base sequences of human RB gene and RB mRNA translated to the protein are registered in a database named GenBank/NCBI under an accession number NM_000321. An amino acid sequence of the protein is registered in a database named GenPept (NCBI Protein Database) under an accession number NP_000312.

p14^{ARF} is a splicing variant of p16^{ink4a} and both are encoded by CDKN2A gene. p14^{ARF} has a function of suppressing degradation of p53 by binding to a p53 stabilizing protein MDM2 and is thus known as, like p16^{ink4a}, one of cancer inhibitory factors.

Base sequences of human p14^{ARF} gene and p14^{ARF} mRNA translated to the protein are registered in a database named GenBank/NCBI under an accession number NM_058195. An amino acid sequence of the protein is registered in a database named GenPept (NCBI Protein Database) under an accession number NP_478102.

CD47 (also referred to as integrin-associated protein (IAP)) is a transmembrane protein belonging to an immunoglobulin superfamily. It is known that CD47 expressed on a cell membrane in a normal cell binds to signal regulatory protein α (SIRPα) on a macrophage cell membrane and thereby inhibits phagocytosis of the macrophage. It is considered to be preferable that cells used in cell transplantation therapy express CD47 to avoid phagocytosis by macrophages. Further, it is reported that CD47 enhances homing and a tissue repair effect of MSCs (Int J Clin Exp Pathol. 2015; 8(9): 10555-10564), thus it is speculated that CD47 has a function of enhancing a therapeutic effect of MSCs on a disease.

Base sequences of human CD47 gene and CD47 mRNA translated to the protein are registered in a database named GenBank/NCBI under accession numbers NM_001777 and NM_198793, respectively. An amino acid sequence of the protein is registered in a database named GenPept (NCBI Protein Database) under an accession number NP_001768.

It is unexpectedly found that, as a result of analysis of gene expression profiles of the activated MSCs, the activation treatment causes a change in expression of p16^{ink4a}-RB pathway factors, specifically, for example, an increase in a gene expression level of p16^{ink4a}, as well as a reduction in gene expression levels of CDK4, CDK6, and RB, which are downstream factors of p16^{ink4a}. Further, a gene expression level of p14^{ARF}, like p16^{ink4a}, is also increased, while a gene expression level of CD47 is decreased. Further, it is experimentally confirmed that the above expression changes of the p16^{ink4a}-RB pathway factors, p14^{ARF}, and CD47 do not occur in MSCs of which a therapeutic effect is not enhanced by the activation treatment.

The present inventors have confirmed that the expression levels of genes related to a therapeutic effect of MSCs, such as OCT4, SOX2, and Nanog, which are factors involved in sternness, and IDO and TSG-6, which are factors involved in immunoregulation/anti-inflammatory function, are increased in the activated MSCs. The present inventors have further confirmed that the gene expression of p16^{ink4a} has a very high positive correlation with the expression of these genes. The gene expression of p16^{ink4a} also has a positive correlation with the gene expression of p14^{ARF}.

Further, it is observed that the expression levels of p16^{ink4a} and p14^{ARF}, like the above genes related to therapeutic effect, are increased in MSCs having a high therapeutic effect, while the expression level of CD47 is decreased in MSCs having a high therapeutic effect. Further, it is experimentally confirmed that a therapeutic effect is decreased both in MSCs deficient in p16^{ink4a} and p14^{ARF} and in MSCs in which CD47 is forcibly expressed.

On the basis of these results, p16^{ink4a} and p14^{ARF} can be used as a marker for evaluating a therapeutic effect of MSCs, which has a positive correlation with a therapeutic effect of the MSCs on a disease, while CDK4, CDK6, and RB, which are downstream factors of p16^{ink4a}, as well as CD47, can be used as a marker for evaluating a therapeutic effect of MSCs, which has a negative correlation with a therapeutic effect of the MSCs on a disease. Further, by using these markers, the therapeutic effect of the MSCs can be evaluated.

Another aspect of the present invention relates to a method for determining a therapeutic effect of MSCs, including: a measurement step of measuring a gene or protein expression level of at least one selected from the group consisting of p16^{ink4a}, p14^{ARF}, CDK4, CDK6, RB, and CD47 in test MSCs; a comparison step of comparing the measured gene or protein expression level with a gene or protein expression level in control MSCs; and a determination step of determining the test MSCs to have a higher therapeutic effect than the control MSCs if the gene or protein expression level of at least one selected from the group consisting of p16^{ink4a} and p14^{ARF} is higher in the test MSCs than in the control MSCs and/or if the gene or protein expression level of at least one selected from the group consisting of CDK4, CDK6, RB, and CD47 is lower in the test MSCs than in the control MSCs.

The method for determining a therapeutic effect of the above aspect includes a measurement step for measuring a gene or protein expression level of at least one, preferably two, three, or all selected from the group consisting of p16^{ink4a}, p14^{ARF}, CDK4, CDK6, RB, and CD47 (hereinafter also referred to as therapeutic effect markers) in test MSCs.

The test MSCs can be any MSCs of which a therapeutic effect is desired to be determined. Examples of the test MSCs include MSCs planned to be used in a cell transplantation therapy. In a case where MSCs are planned to be used in an autologous transplantation therapy, the test MSCs are separated from a subject being to undergo the autologous transplantation therapy, preferably the test MSCs are derived from bone marrow. Determining therapeutic effects of these MSCs prior to transplantation makes it possible to avoid unnecessary cell transplantation to a patient and increase a success rate of a cell transplantation therapy. In a particular embodiment, the test MSCs may be MSCs subjected to a treatment for enhancing a therapeutic effect, and examples of such MSCs include MSCs cultured in a medium containing an activator, that is, MSCs subjected to an activation treatment, and MSCs cultured using a cell culture carrier having a three-dimensional structure formed of a fiber.

A measurement of a gene or protein expression level of a therapeutic effect marker in MSCs can be performed by employing various methods known to those skill in the art, capable of measuring an expression level of a gene or a protein of which a base sequence or an amino acid sequence is known.

A measurement of an expression level of a marker protein can be performed using an antibody specific to the marker protein by a well-known method such as an ELISA method including a direct competitive ELISA, an indirect competitive ELISA, and a sandwich ELISA, an RIA method, in situ hybridization, an immunoblotting analysis, a western blotting analysis, and a tissue array analysis. In this case, the specific antibody is not limited to an animal species from which the specific antibody originates. Further, the specific antibody may be polyclonal or monoclonal, and may be an antibody of a full-length immunoglobulin, a partial fragment such as a Fab fragment and a F(ab')2 fragment, or the like. The specific antibody may be labeled with a fluorescent substance (e.g., FITC, rhodamine, phalloidin, etc.), a colloidal particle such as gold, a fluorescent microbead such as Luminex (registered trademark, Luminex Corp.), heavy metal (e.g., gold, platinum, etc.), a chromoprotein (e.g., phycoerythrin, phycocyanin, etc.), a radioisotope (e.g., ³H, ¹⁴C, ³²P, ³⁵S, ¹²⁵I, and ¹³¹I), an enzyme (e.g., peroxidase, alkaline phosphatase, etc.), biotin, streptavidin, or other labelling compounds.

A measurement of an expression level of a marker gene can be performed by known methods capable of detecting expression of the marker gene, such as a PCR method using nucleic acid primers having an appropriate base sequence designed on the basis of the base sequence of the marker gene, in particular, a real-time PCR method and a digital PCR method allowing absolute quantification of nucleic acids, a hybridization method using a nucleic acid probe having a base sequence capable of hybridizing to the mRNA base sequence of the marker gene under a stringent condition, a microarray method using a chip on which a nucleic acid probe having a base sequence capable of hybridizing to the mRNA base sequence of the marker gene is fixed, and an RNA sequencing method. The nucleic acid primers or the nucleic acid probe may be labeled with a fluorescent substance, a radioisotope, an enzyme, biotin, streptavidin, or other labelling compounds depending on the method used.

The method for determining a therapeutic effect of the above aspect also includes a comparison step for comparing the measured expression level with the expression level in the control MSCs. The control MSCs described herein are MSCs serving as a standard for determining the therapeutic effect. For example, in a case where the test MSCs are planned to be used in a cell transplantation therapy, as the control MSCs, MSCs having the lowest level of the therapeutic effect usable in the cell transplantation therapy can be used. The expression level of the gene or the protein of the therapeutic effect marker in the control MSCs may be simultaneously measured with that in the test MSCs or previously measured.

The method for determining a therapeutic effect of the above aspect further includes a determination step of determining the test MSCs to have a higher therapeutic effect than the control MSCs if the gene or protein expression level of at least one selected from the group consisting of p16^{ink4a} and p14^{ARF} is higher in the test MSCs than in the control MSCs and/or if the expression level of at least oneselected from the group consisting of CDK4, CDK6, RB, and CD47 is lower in the test MSCs than in the control MSCs.

In the determination step, in a case where the therapeutic effect marker measured in the measurement step is p16^{ink4a} or p14^{ARF}, the test MSCs can be determined to have a higher therapeutic effect than the control MSCs if the gene or protein expression level of p16^{ink4a} or p14^{ARF} is higher in the test MSCs than in the control MSCs. Further, in a case where the therapeutic effect marker measured in the measurement step is CDK4, CDK6, RB, or CD47, the test MSCs can be determined to have a higher therapeutic effect than the control MSCs if these gene or protein expression levels are lower in the test MSCs than in the control MSCs.

A cluster analysis can be used in a case where a therapeutic effect of MSCs is determined using a plurality of therapeutic effect markers. Specifically, relative expression data of each therapeutic effect marker measured in the test MSCs, as well as relative expression data of each therapeutic effect marker measured in MSCs of positive control having a high therapeutic effect, that is, MSCs having a sufficient level of the therapeutic effect to be used in a cell transplantation therapy, and relative expression data of each therapeutic effect marker measured in MSCs of negative control having a low therapeutic effect, that is, MSCs having an insufficient level of the therapeutic effect to be used in a cell transplantation therapy are subjected to a cluster analysis, and then the therapeutic effect is determined to be high if the test MSCs and the positive control MSCs are in the same cluster, while the therapeutic effect is determined to be low if the test MSCs and the negative control MSCs are in the same cluster.

A cluster analysis can be performed by an appropriate clustering method, for example, a hierarchical method such as Ward's method and a group average method, using a statistical software such as R version 3.0.1 (Various R programming tools for plotting data: gplots).

Further, the present inventors have confirmed that, in addition to the above therapeutic effect markers, expression levels of, for example, DNMT1, Nanog, SOX2, and OCT4 genes serving as an indicator of sternness and TERT gene known to be involved in a telomerase activity are higher in MSCs having a higher therapeutic effect than in untreated MSCs, and, further, expression levels of p53 gene known to be involved in cellular senescence and α-SMA gene known to be involved in cytoskeleton are lower in MSCs having a higher therapeutic effect than in untreated MSCs. Thus, it is possible that in the measurement step, in addition to measuring expression levels of genes or proteins of the above therapeutic effect markers, gene or protein expression levels of:
(A) at least one, preferably two, three, four, five, six, seven, or all selected from the group consisting of DNMT1, Nanog, SOX2, OCT4, IDO, TSG6, IL-6, and TERT; and/or
(B) at least one, preferably all selected from the group consisting of p53 and α-SMA,
are measured, and
in the determination step, the result thus obtained is combined with changes in expression levels of genes or proteins of the above therapeutic effect markers. Specifically, it is possible to determine that the test MSCs have a high therapeutic effect if the gene or protein expression level of (A) is higher in the test MSCs than in the control MSCs and/or if the gene or protein expression level of (B) is lower in the test MSCs than in the control MSCs, in addition to the higher gene or protein expression level of at least one selected from the group consisting of p16^{ink4a} and p14^{ARF} in the test MSCs than in the control MSCs and/or the lower gene or protein expression level of at least one selected from the group consisting of CDK4, CDK6, RB, and CD47 in the test MSCs than in the control MSCs.

Further, the present invention includes the method in which, in the above-described method of the above aspect for determining the therapeutic effect, a proportion of CD47 positive cells in the MSCs is measured instead of the gene or protein expression level of CD47 in the measurement step; and the test MSCs are determined to have the higher therapeutic effect than the control MSCs if the proportion of CD47 positive cells is lower in the test MSCs than in the control MSCs instead of the lower gene or protein expression level of the CD47 in the test MSCs than in the control MSCs in the determination step.

A proportion of CD47 positive cells in MSCs can be determined by labeling the MSCs with labeled antibodies against CD47 and analyzing the labelled cells by a flow cytometry method or the like using isotype controls or the like as a negative control.

### Method for Determining Suitability of MSCs to be treated with Treatment for Enhancing Therapeutic Effect

A further aspect of the present invention relates to a method for determining suitability of MSCs to be treated with a treatment for enhancing a therapeutic effect of MSCs, including: a measurement step of measuring a gene or protein expression level of at least one selected from the group consisting of p16^{ink4a}, p14^{ARF}, CDK4, CDK6, RB, and CD47 in treated MSCs; a comparison step of comparing the measured gene or protein expression level with a gene or protein expression level in untreated MSCs; and a determination step of determining the MSCs to be suitable to be treated with the treatment if the gene or protein expression level of at least one selected from the group consisting of p16^{ink4a} and p14^{ARF} is higher in the treated MSCs than in the untreated MSCs and/or if the gene or protein expression level of at least one selected from the group consisting of CDK4, CDK6, RB, and CD47 is lower in the treated MSCs than in the untreated MSCs.

A still further aspect of the present invention relates to a method for determining suitability of MSCs to be treated with a treatment for enhancing a therapeutic effect of MSCs, including: a measurement step of measuring a gene or protein expression level of at least one selected from the group consisting of p16^{ink4a}, p14^{ARF}, CDK4, CDK6, RB, and CD47 in treated MSCs; a comparison step of comparing the measured gene or protein expression level with a gene or protein expression level in untreated MSCs; and an exclusion determination step of determining the MSCs to be not suitable to be treated with the treatment if the gene or protein expression level of at least one selected from the group consisting of p16^{ink4a} and p14^{ARF} is lower or equal in the treated MSCs to the untreated MSCs and/or if the gene or protein expression level of at least one selected from the group consisting of CDK4, CDK6, RB, and CD47 is higher or equal in the treated MSCs to in the untreated MSCs.

The term "treated MSCs" in the suitability determination method of the above aspect refers to MSCs subjected to a certain treatment for enhancing a therapeutic effect of MSCs. Further, the term "untreated MSCs" refers to MSCs not subjected to such a treatment or MSCs subjected to a control treatment of such a treatment. Examples of such a treatment and a control treatment thereof include cultivation in the presence of an activator/cultivation in the absence of an activator, cultivation on a three-dimensional culture carrier/cultivation on a two-dimensional culture carrier, cultivation at a low oxygen concentration or in a low nutritional state/cultivation at a normal oxygen concentration or in a normal nutritional state, cultivation in a low-gravity environment or an excessive gravity environment/cultivation in a normal gravity environment, cultivation in the presence of a factor including growth factor such as bFGF, TNF-α, IL-6, TGF-β or PDGF, or cytokine/chemokine, or the like/cultivation in the absence of those factors, and transgenic treatment by gene transfer/no treatment.

As described above, p16^{ink4a} and p14^{ARF} are positively correlated with a therapeutic effect of MSCs, and CDK4, CDK6, RB, and CD47 are negatively correlated with a therapeutic effect of MSCs, thus, comparing expression levels of these factors between MSCs subjected to a treatment for enhancing the therapeutic effect of MSCs and MSCs not subjected to the treatment makes it possible to determine whether the MSCs are responsive to the treatment and whether the MSCs have suitability to be treated with the treatment.

The measurement step and comparison step in the suitability determination method of the above embodiment are as described in the corresponding steps in the above therapeutic effect determination method. Further, an expression level of a gene or protein of the therapeutic effect marker in the untreated MSCs may be simultaneously measured with that of a gene or protein of the therapeutic effect marker in the treated MSCs or previously prepared.

If the gene or protein expression level of at least one selected from the group consisting of p16^{ink4a} and p14^{ARF} is higher in the treated MSCs than in the untreated MSCs and/or if the gene or protein expression level of at least one selected from the group consisting of CDK4, CDK6, RB, and CD47 is lower in the treated MSCs than in the untreated MSCs, it can be predicted that the therapeutic effect of the MSCs is enhanced by the treatment and it can be determined that the MSCs are suitable to be treated with the treatment, in other words, the MSC has suitability to be treated with the treatment. The suitability determination method of the above aspect can also be expressed as a method for determining an enhancement in a therapeutic effect of MSCs by a treatment for enhancing a therapeutic effect of MSCs, the method including the above measurement step and comparison step, as well as a step in which, if the gene or protein expression level of at least one selected from the group consisting of p16^{ink4a} and p14^{ARF} is higher in the treated MSCs than in the untreated MSCs and/or if the gene or protein expression level of at least one selected from the group consisting of CDK4, CDK6, RB, and CD47 is lower in the treated MSCs than in the untreated MSCs, it is determined that the therapeutic effect of the MSCs is enhanced by the treatment.

On the other hand, if the gene or protein expression level of at least one selected from the group consisting of p16^{ink4a} and p14^{ARF} in the treated MSCs is lower than or equal to that in the untreated MSCs and/or if the gene or protein expression level of at least one selected from the group consisting of CDK4, CDK6, RB, and CD47 in the treated MSCs is higher than or equal to that in the untreated MSCs, it can be predicted that the therapeutic effect of the MSCs is unlikely to be enhanced by the treatment for enhancing a therapeutic effect and it can be determined that the MSCs have low or no suitability to be treated with the treatment. The suitability determination method of the above embodiment can also be expressed as a method for determining non-existence of an enhancement in a therapeutic effect of MSCs by a treatment for enhancing a therapeutic effect of MSCs, the method including the above measurement step and comparison step, as well as a step in which, if the gene or protein expression level of at least one selected from the group consisting of p16^{ink4a} and p14^{ARF} is lower or equal in the treated MSCs to the untreated MSCs and/or if the gene or protein expression level of at least one selected from the group consisting of CDK4, CDK6, RB, and CD47 is higher or equal in the treated MSCs to the untreated MSCs, it is determined that the therapeutic effect of the MSCs is not enhanced by the treatment.

In the suitability determination method of the above aspect, as in the therapeutic effect determination method described above, a cluster analysis can be used for determining the suitability by using a plurality of therapeutic effect markers; factors involved in sternness, factors involved in immunoregulation/anti-inflammatory function, and factors involved in a telomerase activity can be used in combination for the determination; and a proportion of CD47 positive cells, instead of a gene or protein expression level of CD47, in MSCs can be used. Those details are as described in the therapeutic effect determination method described above.

Note that, instead of the treatment for enhancing a therapeutic effect of MSCs in the suitability determination method of the above aspect, a treatment with which it is unknown whether a therapeutic effect of MSCs is enhanced can be performed, and, then, if the gene or protein expression level of at least one selected from the group consisting of p16^{ink4a} and p14^{ARF} is higher in the treated MSCs than in the untreated MSCs and/or if the gene or protein expression level of at least one selected from the group consisting of CDK4, CDK6, RB, and CD47 is lower in the treated MSCs than in the untreated MSCs, it can be determined that the treatment is applicable for enhancing a therapeutic effect of MSCs. The present invention also provides such a determination method.

In the therapeutic effect determination method and the suitability determination method described above, if the MSCs are determined to have a high therapeutic effect or high suitability to be treated with the treatment for enhancing a therapeutic effect, or the therapeutic effect is determined to be enhanced in the MSCs, production or investigation of MSCs for transplanting with the treatment may be further proceeded. On the other hand, if the MSCs are determined to have a low therapeutic effect or low suitability, or the therapeutic effect is determined not to be enhanced in the MSCs, production of MSCs for transplanting with the treatment may not be proceeded, making it possible to reduce a consumption of MSCs and agents required for the treatment such as an activator.

### Cell Preparation

Another aspect of the present invention relates to a cell preparation including MSCs having a proportion of CD47 positive cells of 2% or less.

As described above, CD47 has been confirmed to be a therapeutic effect marker having a negative correlation with a therapeutic effect of MSCs. The present inventors have found that MSCs having a very low proportion of CD47 positive cells can be produced by the production method of the first aspect. Such MSCs are expected to have a very high therapeutic effect, thus a cell preparation including such MSCs can be used as a pharmaceutical for treating and/or preventing a disease in which a cell transplantation therapy using MSCs is effective.

The cell preparation can be produced using MSCs produced by the production method of the first aspect. The MSCs included in the cell preparation are characterized by having a proportion of CD47 positive cells of 2% or less. As shown in Example below, considering that even MSCs collected from a young person expected to have a high therapeutic effect have a proportion of CD47 positive cells of more than 2%, the MSCs having such a very low proportion of CD47 positive cells can be expected to have an unprecedentedly high therapeutic effect.

A proportion of CD47 positive cells in the MSCs included in the cell preparation is 2% or less, preferably 1.5% or less. A proportion of CD47 positive cells in the MSCs can be confirmed by the procedure previously described in the above therapeutic effect determination method.

The cell preparation can also be produced by concentrating CD47 negative cells from an MSC population such as MSCs separated from a subject. Thus, the present invention also provides a method for producing a cell preparation including MSCs, the method including a step of concentrating the CD47 negative MSCs from an MSC population.

Concentration of the CD47 negative MSCs can be performed by a well-known technique used for concentrating cells not having a specific cell surface marker, for example, a negative selection of CD47. In the negative selection of CD47, an MSC population is subjected to beads to which CD47 specific antibodies are bound or flow cytometry using CD47 specific antibodies, and MSCs not bound to the antibodies are recovered.

The cell preparation includes an effective amount of MSCs. The term "effective amount" means an amount which is effective for treating and/or preventing a disease. Such an effective amount is appropriately adjusted depending on a type of disease, the severity of symptom, an administration route, a patient, or other medical factors. In a preferred embodiment, the effective amount of MSCs is from 10⁴ cells to 10⁹ cells, preferably from 10⁵ cells to 10⁸ cells, per kg of weight of an individual to be administered in a case of systemic administration, while the effective amount of MSCs is from 10² cells to 10⁹ cells, preferably from 10⁴ cells to 10⁶ cells, per kg of weight of an individual to be administered in a case of local administration. As described above, the MSCs produced in the production method according to the first aspect of the present invention have a higher therapeutic effect as compared with the MSCs activated by using an activator on a culture carrier not having a three-dimensional structure formed of a fiber, typically, on a two-dimensional culture carrier, thus the effective amount of MSCs in the cell preparation can be from 1/20 to 1/2, preferably from 1/10 to 1/4, of the effective amount of the activated MSCs produced using the cell culture carrier not having a three-dimensional structure formed of a fiber. The cell preparation having such an effective amount can be administered once or over a plurality of times.

Further, a culture product of MSCs contains various humoral factors produced by the MSCs, and the humoral factors are also known to have a therapeutic effect on a disease. Thus, a culture product obtained by cultivating the MSCs included in the cell preparation of the above aspect can also be used as a pharmaceutical for treating and/or preventing a disease. The culture product is preferably a culture supernatant and can be obtained by cultivating MSCs in the presence of an activator or cultivating activated MSCs in a medium normally used for cultivation of MSCs, for example, α-MEM, DMEM, or the like. An effective amount of the culture product in a pharmaceutical including the MSC culture product is from 0.01 mg to 100 mg, preferably from 0.02 mg to 50 mg, more preferably from 0.05 mg to 20 mg, per kg of weight of an individual to be administered, and this amount can be administered once or over a plurality of times.

The cell preparation and the pharmaceutical including the culture product (hereinafter collectively referred to as a pharmaceutical of the present invention) described above are normally used in the form of parenteral preparation such as an injection. Examples of a carrier that can used in the parenteral preparation include an aqueous carrier such as a physiological saline solution and an isotonic solution containing glucose, D-sorbitol, and the like. Further, the pharmaceutical of the present invention may be a pharmaceutical composition containing a buffer, a stabilizer, a preservative, and other components, which are pharmaceutically acceptable.

A method for administering the pharmaceutical of the present invention is not particularly limited. In a case of the parenteral preparation, examples of the administering method include local administration such as intravascular administration (preferably intravenous administration), intraperitoneal administration, intestinal administration, subcutaneous administration, and subcapsular administration (a capsule is a membrane tissue that covers various organs). In a preferred embodiment, the pharmaceutical of the present invention is administered to a living body by intravenous administration. Further, the pharmaceutical of the present invention may be used in combination with other pharmaceuticals depending on a disease to be treated and/or prevented.

The cell preparation can also be locally administered to a living body by attaching the cell preparation in the form of being adhered to the three-dimensional culture carrier to a site where the disease occurs, a site where the disease may occur, a site that causes the disease, or the vicinity thereof in the living body. In the present embodiment, a material forming the fiber of the three-dimensional culture carrier is preferably a biocompatible material, particularly preferably a biodegradable polymer.

The pharmaceutical of the present invention can be used to treat and/or prevent a disease in which a cell transplantation therapy using MSCs is effective, for example, diabetes and its complications, a cerebrovascular disease, a degenerative brain disease, a demyelinating disease, a functional seizure disease, dementia, a peripheral nerve disease, a cardiovascular disease, an autoimmune disease, a liver/biliary tract/pancreatic disease, a stomach/duodenum disease, a small intestine/large intestine disease, a thyroid disease, a blood/hematopoietic disease, a pulmonary disease, an acute kidney disease and a chronic kidney disease, an ocular disease, a skin disease, a muscle/bone disease, and an injury and a graft-versus-host disease (GVHD). Specific examples of these diseases include type 1 diabetes and type 2 diabetes, and their complications, for example, diabetic nephropathy, diabetic retinopathy, diabetic neuropathy, diabetic gangrene, and the like; a cerebrovascular disease such as stroke (cerebral infarction and cerebral hemorrhage) and a degenerative brain disease such as Parkinson's disease, Huntington's disease, corticobasal degeneration, multiple system atrophy, spinocerebellar degeneration, and amyotrophic lateral sclerosis; a demyelinating disease such as multiple sclerosis, acute disseminated encephalomyelitis, and neuromyelitis optica spectrum disorder; a functional seizure disease such as epilepsy and cerebral palsy; dementia such as vascular dementia, Alzheimer's disease, Lewy body dementia, frontotemporal dementia, and diabetes-related dementia; a peripheral nerve disease such as Guillain-Barre syndrome, peripheral neuropathy, facial paralysis, trigeminal neuralgia, micturition disorder, erectile dysfunction, and dysautonomia; a cardiovascular disease such as myocardial infarction, angina, arteriosclerosis obliterans, and cardiomyopathy; an autoimmune disease such as rheumatoid arthritis, systemic lupus erythematosus, Sjogren's syndrome, polymyositis, dermatomyositis, scleroderma, mixed connective tissue disease, polymyalgia rheumatica, eosinophilia, Behçet's disease, sarcoidosis, Still's disease, spondyloarthritis, and Kawasaki disease; a liver/biliary tract/pancreatic disease such as acute/chronic hepatitis, liver cirrhosis, autoimmune hepatitis, primary biliary cholangitis, primary sclerosing cholangitis, acute/chronic pancreatitis, and autoimmune pancreatitis; a stomach/duodenum disease such as acute/chronic gastritis and gastric/duodenum ulcer; a small intestine/large intestine disease such as Crohn's disease, ulcerative colitis, ischemic colitis, and irritable bowel syndrome; a thyroid disease such as Basedow's disease and acute/chronic thyroiditis; a blood/hematopoietic disease such as autoimmune hemolytic anemia, polycythemia vera, and idiopathic thrombocytopenic purpura; a pulmonary disease such as chronic obstructive pulmonary disease, interstitial lung disease, pulmonary fibrosis, pneumoconiosis, bronchial asthma, eosinophilic pneumonia, and acute respiratory distress syndrome (ARDS); an acute kidney disease such as an acute kidney disease associated with reduced body fluid or ischemia, ANCA-associated nephritis, microscopic polyangiitis, granulomatosis with polyangiitis, eosinophilic granulomatosis with polyangiitis, malignant hypertension, cryoglobulinemia, postinfectious glomerulonephritis, IgA nephropathy, acute interstitial nephritis, drug-induced kidney injury, myeloma kidney, gouty kidney, rhabdomyolysis, and acute tubular necrosis; a chronic kidney disease such as membranous nephropathy, membranoproliferative glomerulonephritis, minimal change nephritic syndrome, focal glomerulosclerosis, lupus nephritis, amyloidosis, nephrosclerosis, purpura nephritis, IgG4-related kidney disease, Sjögren's syndrome, scleroderma kidney disease, chronic interstitial nephritis, and polycystic kidney disease; an ocular disease such as macular degeneration, optic neuritis, and uveitis; a skin disease such as atopic dermatitis, bullous disease, and Stevens-Johnson syndrome; a muscle/bone disease such as myasthenia gravis, muscular dystrophy, osteoarthritis of hip, osteonecrosis of femoral head, osteoporosis, and carpal tunnel syndrome; an injury such as spinal cord injury and cerebral contusion; a wound such as bedsore, mouth ulcer, graft-versus-host disease (GVHD), anastomotic leakage, and contusion of an organ. A disease to which the pharmaceutical of the present invention is preferably applied is diabetic nephropathy, an acute kidney disease, a chronic kidney disease, diabetic retinopathy, diabetic neuropathy, diabetic gangrene, Alzheimer's disease, diabetes-related dementia, rheumatoid arthritis, polymyositis, and a wound.

The treatment and/or prevention used in the present specification include all types of pharmaceutically acceptable therapeutic and/or preventive interventions intended for curing, temporary remission, prevention, or the like of a disease or a symptom. That is, the treatment and/or prevention of a disease or a symptom include pharmaceutically acceptable interventions for various purposes including delaying or stopping the progress of a disease or a symptom, causing a lesion to regress or disappear, preventing onset or recurrence of a disease, and the like.

The present invention also includes, as another aspect, a method for treating and/or preventing a disease in which a cell transplantation therapy using MSCs is effective, the method including administration of the pharmaceutical of the present invention in an effective amount to a subject in need thereof. Meaning of each term in the present aspect is as described above.

The present invention will be described more in detail by the following examples. The present invention is not limited to these examples.

### Examples

### Example 1 Preparation of MSCs Subjected to Activation Treatment on Three-Dimensional Culture Carrier

### 1) Preparation of Activator

In accordance with the description in Patent Literature 1, an activator was prepared from a human placental tissue. In brief, 100 mL of a serum free medium (alpha-MEM) was added to 50 g wet weight of a finely stripped human placental tissue, followed by shaking at 4°C for 72 hours. A supernatant was recovered by centrifugal separation to obtain an activator as a placental tissue extract.

### 2) Activation Treatment

MSCs derived from bone marrow collected from a patient with osteoarthritis of hip at the time of the artificial joint replacement surgery (OA-MSCs) were cultured in an MSC culture medium (DMEM containing 15% FBS, 1% penicillin, 1% streptomycin, and 100 mg/dL glucose) without the activator. The cells were recovered and seeded on three-dimensional culture carriers, namely, Preset VECELL 6-well (registered trademark) (Vessel Inc.) at 8 × 10⁴ cells/well, Cellbed 24-well (registered trademark) (Japan Vilene Company) at 2 × 10⁴ cells/well, and 3D-insert PS-200 12-well and 3D-insert PS-400 12-well (3D Biotek LLC.) each at 3.8 × 10⁴ cells/well, followed by cultivation using the MSC culture medium at 37°C for 72 hours. After removing the medium, the MSC culture medium containing the activator described above at a concentration converted in terms of protein of 10 µg/mL, 1 µg/mL, or 0.1 µg/mL, or the MSC culture medium not containing the activator was added to Preset VECELL 6-well in an amount of 2 mL, Cellbed 24-well in an amount of 0.6 mL, and 3D-insert PS-200 12-well and 3D-insert PS-400 12-well each in an amount of 1 mL, followed by cultivation at 37°C for four days. After performing this cultivation from one to three times, cells were recovered to prepare OA-MSCs having the passage number of from 1 to 3.

Further, cells were seeded on a flat plate-like two-dimensional cell culture carrier, namely, Corning (registered trademark) Costar (registered trademark) cell culture 6-well plate (Thermo Fisher Scientific) at 6 × 10⁴ cells/well, followed by cultivation in the presence or absence of the activator at a concentration of 100 µg/mL. By repeating this cultivation one more time, control OA-MSCs having the passage number of 2 were prepared.

### Example 2 Gene Expression Analysis and Morphological Observation of OA-MSCs Subjected to Activation Treatment on Three-Dimensional Culture Carrier

### 1) Gene Expression Analysis

Total RNAs were extracted from each of the OA-MSCs prepared in 2) of Example 1 using Tri Reagent (Molecular Research Center, Inc.). Clone DNAs were synthesized by a reverse transcription reaction, and real-time PCR was performed on OCT4, Nanog, SOX2, DNMT1, TERT, IL-6, IDO, TSG-6, p21, p53, α-SMA, and 18s RNA using primer sets having base sequences shown in Table 1. In Table, (F) represents a forward primer, while (R) represents a reverse primer.

**[Table 1]**

| Gene | Base Sequence | SEQ ID NO: |
|---|---|---|
| OCT4(F) | GACAGGGGAGGGGAGGAG | 1 |
| OCT4(R) | CTTCCCTCCAACCAGTTGCCC | 2 |
| NANOG(F) | TGGACACTGGCTGAATCCTTC | 3 |
| NANOG(R) | CGTTGATTAGGCTCCAACCAT | 4 |
| SOX2(F) | GGGAAATGGGAGGGGTGCAA | 5 |
| SOX2(R) | TTGCGTGAGTGTGGATGGGA | 6 |
| DNMT1(F) | CGTAAAGAAGAATTATCCGAGG | 7 |
| DNMT1(R) | GTTTTCTAGACGTCCATTCAC | 8 |
| TERT(F) | CGGAAGAGTGTCTGGAGC | 9 |
| TERT(R) | GGATGAAGCGGAGTCTGGA | 10 |
| IL-6(F) | GATGAGTACAAAAGTCCTGATCCA | 11 |
| IL-6(R) | CTGCAGCCACTGGTTCTGT | 12 |
| IDO(F) | GCATTTTTCAGTGTTCTTCGCATA | 13 |
| IDO(R) | TCATACACCAGACCGTCTGATAGC | 14 |
| TSG-6(F) | CCCATTGTGAAGCCAGGGCCCAACTG | 15 |
| TSG-6(R) | GGAAGCTCATCTCCACAGTATCTTCCC | 16 |
| P16INK4a(F) | AGCATGGAGCCTTCGGCTGA | 17 |
| P16INK4a(R) | CCATCATCATGACCTGGATCG | 18 |
| RB(F) | GCTAGCCTATCTCCGGCTAAA | 19 |
| RB(R) | CTGGAAAAGGGTCCAGATGA | 20 |
| P21(F) | GAGACTCTCAGGGTCGAAAA | 21 |
| P21(R) | TTAGGGCTTCCTCTTGGAGA | 22 |
| TP53(F) | TGACTGTACCACCATCCACTA | 23 |
| TP53(R) | AAACACGCACCTCAAAGC | 24 |
| alpha-SMA(F) | GCAGCCCAGCCAAGCACTGT | 25 |
| alpha-SMA(R) | TGGGAGCATCGTCCCCAGCA | 26 |
| RNA18S(F) | ATCGGGGATTGCAATTATTC | 27 |
| RNA18S(R) | CTCACTAAACCATCCAATCG | 28 |

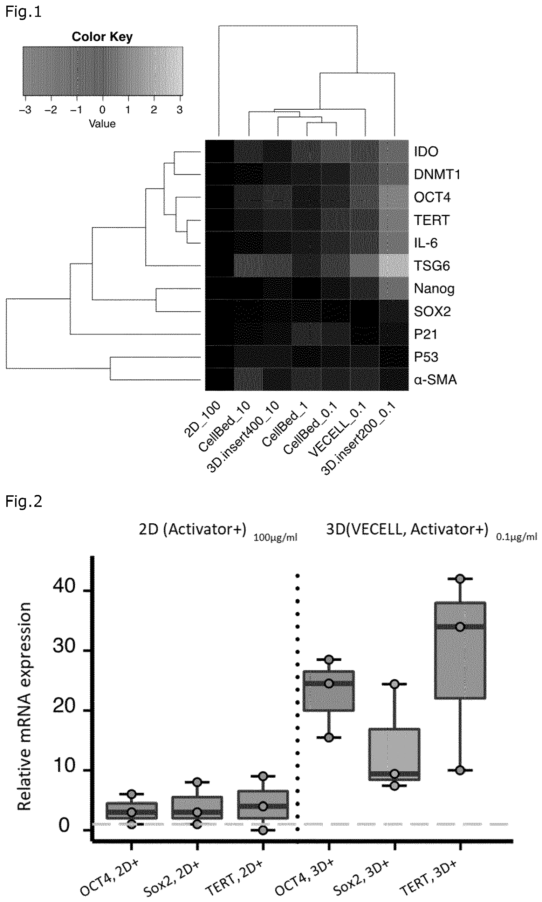

A ΔΔCT value was calculated on the basis of the control OA-MSCs cultured on the two-dimensional culture carrier in the presence of the activator at 100 µg/mL using 18s RNA as a housekeeping gene, and then gene expression profiles were analyzed and clustered. Fig. 1 shows results of OA-MSCs cultured on Preset VECELL in the presence of the activator at 0.1 µg/mL (VECELL_0.1), OA-MSCs cultured on Cellbed in the presence of the activator at 0.1, 1, or 10 µg/mL (CellBed_0.1, CellBed_1, and CellBed_10, respectively), OA-MSCs cultured on 3D-insert PS-200 in the presence of the activator at 0.1 µg/mL (3D. insert200_0.1), OA-MSCs cultured on 3D-insert PS-400 in the presence of the activator at 10 µg/mL (3D. insert400_10), and control OA-MSCs cultured on the two-dimensional culture carrier in the presence of the activator at 100 µg/mL (2D_100). Further, Fig. 2 show relative gene expression levels of OCT4, SOX2, and TERT in 2D_100 and VECELL_0.1 with respect to OA-MSCs cultured on the two-dimensional culture carrier in the absence of the activator (2D_0).

It was confirmed that expression levels of DNMT1, Nanog, SOX2, and OCT4 genes known to be involved in stemness, IDO, TSG6, and IL-6 genes known to be involved in immunoregulation/anti-inflammatory function, and TERT gene known to be involved in a telomerase activity were increased, while expression levels of P53 gene known to be involved in cellular senescence and α-SMA gene known to be involved in cytoskeleton were decreased in any of the OA-MSCs subjected to the activation treatment on the three-dimensional culture carrier in comparison with 2D_100 and 2D_0. This result shows that an expression tendency of a group of genes known as a marker for MSCs having a therapeutic effect is more strongly emphasized in the MSCs subjected to the activation treatment on the three-dimensional culture carrier than in the MSCs subjected to the activation treatment on the two-dimensional culture carrier.

### 2) Morphological Observation

Next, OA-MSCs cultured on Preset VECELL in the presence or absence of the activator at 0.1 µg/mL (MSC(3D·activator+) and MSC(3D·activator-)) and OA-MSCs cultured on the two-dimensional culture carrier in the presence or absence of the activator at 100 µg/mL (MSC(2D·activator+) and MSC(2D·activator-)) were fixed with a 0.1 M phosphate buffer solution containing 2.5% glutaraldehyde and 2% paraformaldehyde at 4°C for 1 hour, followed by fixation with a 0.1 M phosphate buffer solution containing 1% osmium at 4°C for 1 hour. Subsequently, dehydrated and dried cells were subjected to metal coating and observed by a scanning electron microscope.

In the MSCs cultured on the two-dimensional culture carrier, the number of projections was increased, a cell thickness was increased (lower row in Fig. 3), and more vesicles assumed to be exosomes were formed (lower row in Fig. 4) by addition of the activator. The MSCs cultured on the three-dimensional culture carrier without adding the activator showed morphological changes equivalent to or greater than those of the MSCs two-dimensionally cultured in the presence of the activator (top left and bottom right of Fig. 3 and top left and bottom right of Fig. 4). Further, cultivation on the three-dimensional culture carrier in the presence of the activator drastically increased the number of projections of the MSCs to form a network structure (top right in Fig. 3), and also increased vesicle formation (top right in Fig. 4). Note that, in the MSCs (3D·activator+) shown in top right of Fig. 4, traces formed by releasing massively forming vesicles appear as a large number of cavities.

### 3) Gene Expression Analysis and Morphological Observation of OA-MSCs Subjected to Activation Treatment on Other Culture Carrier

A cell culture base material A (average fiber diameter of from 0.1 to 0.5 µm, void ratio of 70% or less, average pore area of 0.2 µm², 24-well) was used as a culture carrier of Comparative example, and an activation treatment of OA-MSCs was performed at an activator concentration of 1 µg/mL or 0.1 µg/mL in the same manner as performed with Cellbed 24-well in 2) of Example 1. The base material A partially has a three-dimensional structure formed of a fiber. However, about 50% of the cell adhesion surface of the base material A is occupied by a flat film-like portion not having a three-dimensional structure.

Further, a cell culture base material B was used as another three-dimensional culture carrier to perform an activation treatment of OA-MSCs. The cell culture base material B is a three-dimensional culture carrier including a nanofiber made of polyglycolic acid on a base member made of polyglycolic acid, and produced by a method described in WO 2016/068266 pamphlet and Liu L, Kamei K et al., Biomaterials 124(2017) 47-54.

OA-MSCs were cultured in an MSC culture medium (DMEM containing 15% FBS, 1% penicillin, 1% streptomycin, and 100 mg/dL glucose) without the activator. Cells were recovered and 8 × 10⁴ MSCs were seeded on the cell culture base material B having a size of 2.5 cm × 2.5 cm which was placed in a two-dimensional culture carrier (Corning (registered trademark) Costar (registered trademark) cell culture 6-well plate, Thermo Fisher Scientific). After adding 2 mL of the MSC culture medium containing the activator at 1 µg/mL, cells were cultured at 37°C for 72 hours to prepare an activation-treated MSC sheet having the passage number of 1. The MSC sheet prepared in this method includes MSCs at about 8,500 cells/cm².

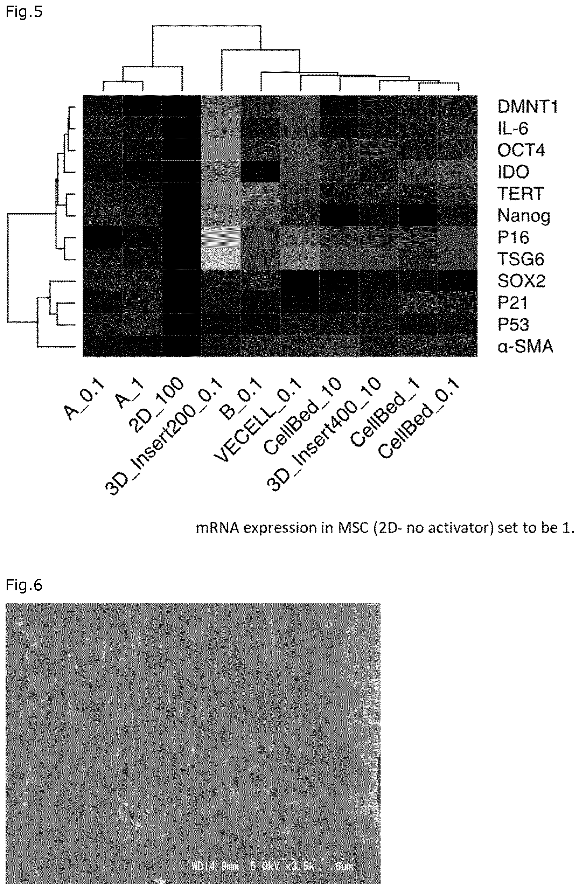

The MSCs were recovered from the base materials A and B, and an expression level of each gene was measured by real-time PCR. The result was combined with the previous real-time PCR result and clustered. The result is shown in Fig. 5. The OA-MSCs subjected to the activation treatment on the base material A showed a gene expression profile similar to that of 2D_100, confirming that the activation treatment on the base material A only caused the same level of the effect as the activation treatment on the two-dimensional culture carrier. Further, the OA-MSCs subjected to the activation treatment on the base material B showed a gene expression profile similar to that obtained with other three-dimensional culture carriers, thus it was speculated that the effect of the activation treatment was similar to that obtained with other three-dimensional culture carriers.

Further, Fig. 6 shows an observation image of the OA-MSCs subjected to the activation treatment on the base material B obtained by a scanning electron microscope. A large number of vesicles were observed as is the case for the OA-MSCs subjected to the activation treatment on Preset VECELL described above.

### Example 3 Gene Expression Analysis of MSCs Derived from Healthy Person Subjected to Activation Treatment on Three-Dimensional Culture Carrier

MSCs derived from bone marrow collected from a 22-year old female having no symptom other than anterior cruciate ligament injury (MSCs derived from healthy person) were cultured in an MSC culture medium without the activator. Cells were recovered and seeded on Preset VECELL 6-well (registered trademark) (Vessel Inc.) at 8 × 10⁴ cells/well, followed by cultivation using the MSC culture medium at 37°C for 72 hours. After removing the medium, 2 mL of the MSC culture medium containing the activator at 1 µg/mL was added to the cells, and cultivation was continued to prepare activation-treated MSCs (3D·activator+) derived from healthy person having the passage number of 1.

Further, MSCs derived from healthy person were seeded on a two-dimensional culture carrier, namely, Corning (registered trademark) Costar (registered trademark) cell culture 6-well plate (Thermo Fisher Scientific) at 6 × 10⁴ cells/well, followed by cultivation in the presence or absence of the activator at a concentration of 100 µg/mL. By repeating this cultivation one more time, MSCs derived from healthy person having the passage number of 2 with or without the activation treatment (2D·activator+ and 2D·activator-) were prepared.

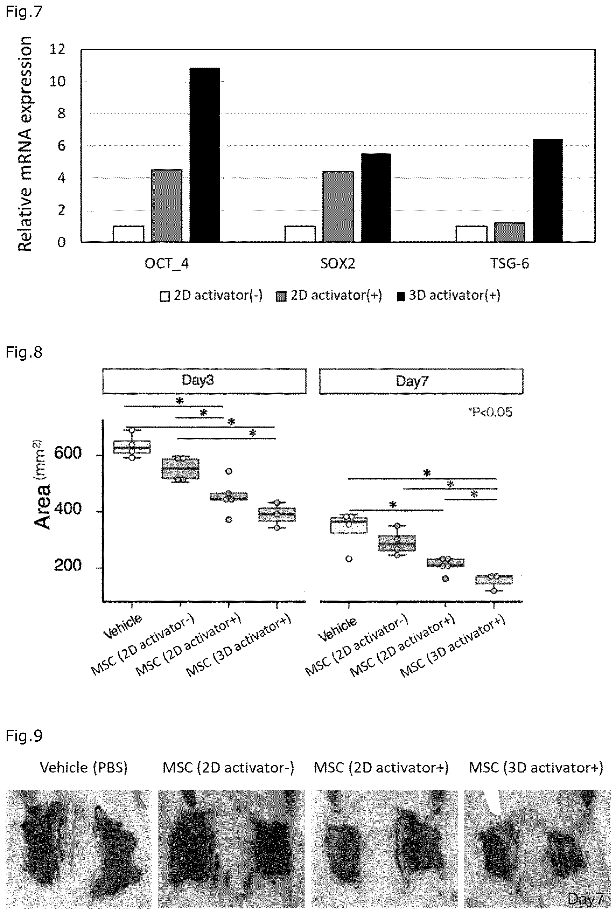

An expression level of each gene in the above three kinds of MSCs was measured by real-time PCR as in 1) of Example 2. Fig. 7 shows relative gene expression levels of OCT4, SOX2, and TSG-6 in MSCs subjected to the activation treatment (2D·activator+ and 3D·activator+) with respect to MSCs not subjected to the activation treatment (2D·activator-). An expression level of each gene was higher in the order of 2D·activator-, 2D·activator+, and 3D·activator+, and, in particular, expression levels of OCT4 and TSG-6 showed significantly higher values in 3D·activator+. This suggests that the activation treatment on the three-dimensional culture carrier can activate not only MSCs derived from a patient with a disease, but also MSCs derived from a healthy person to enhance their therapeutic effect.

### Example 4 Therapeutic Effect of MSCs Subjected to Activation Treatment on Three-Dimensional Culture Carrier (Skin Defect)

Skin defect model rats were made by excising a 3 cm × 3 cm skin in two places on the back of 10-weeks old Lewis rats. One day after the skin excision, MSCs (2D·activator-) (5 × 10⁵ cells/rat), MSCs (2D·activator+) (5 × 10⁵ cells/rat), MSCs (3D·activator+) (1 × 10⁵ cells/rat), prepared in 2) of Example 1, or PBS (vehicle) were each intravenously administered to the rats, followed by keeping for 21 days.

A degree of wound healing was evaluated by measuring a remaining defect area in the skin defect part three days and seven days after the skin excision. The result is shown in Fig. 8. Further, Fig. 9 shows photographs of the skin defect part taken seven days after the skin excision. It was confirmed that the wound was healed earlier in the rats to which the MSCs (3D·activator+) were administered as compared with the rats to which the MSCs (2D·activator+) were administered, despite the number of the administered cells being 1/5.

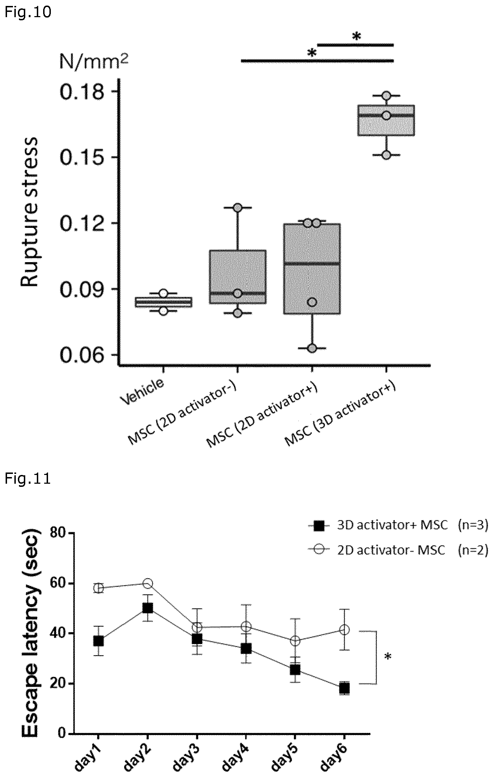

Further, 21 days after the skin excision, a skin in a size of 2 cm × 2 cm at a periphery of the skin defect part was cut out from each rat, and the rupture stress of the skin was measured using a precision universal tester (AG-1) (Shimadzu Corp.). The result is shown in Fig. 10. It was confirmed that the skin tissues in the rats to which the MSCs (3D·activator+) were administered acquired the higher rupture stress as compared with the skin tissues in the rats to which the MSCs (2D·activator+) were administered, despite the number of the administered cells being 1/5.

On the basis of the concentration of the activator used for the activation in Example 1, the number of human patients who could undergo treatment or the like with the MSCs activated by using the activator produced from one human placenta was calculated. As a result, it was estimated that about 10,000 patients could be treated in a case where the activation was performed using the cell culture carrier having a three-dimensional structure formed of a fiber, while about 10 patients could be treated in a case where the activation was performed using the cell culture carrier not having a three-dimensional structure formed of a fiber.

Further, a high therapeutic effect of the MSCs activated using the cell culture carrier having a three-dimensional structure formed of a fiber as confirmed in Example 4 can significantly reduce the number of the cells required for the treatment and/or prevention, considering their high therapeutic effect as well as the fact that this high effect was obtained by 1/5 of the number of the administered cells of the MSCs activated using the two-dimensional culture carrier not having a three-dimensional structure formed of a fiber. This provides the advantage of relieving a treatment burden on a patient, for example, by shortening administration time and reducing a side effect. Further, this can reduce the number of passage times in subculture for achieving the required cell number, providing the advantage of shortening a cell production period, reducing costs, and the like.

### Example 5 Therapeutic Effect of MSCs Activated on Three-Dimensional Culture Carrier (Cognitive Function)

OA-MSCs were seeded on Preset VECELL 6-well (registered trademark) (Vessel Inc.) at 8 × 10⁴ cells/well, followed by cultivation using the MSC culture medium at 37°C for 72 hours. After removing the medium, an MSC culture medium containing the activator at 0.1 µg/mL was added to the cells in an amount of 2 mL/well. After cultivation was performed at 37°C for four days, the cells were recovered to prepare activation-treated OA-MSCs (3D·activator+) having the passage number of 1. Further, the OA-MSCs were seeded on a two-dimensional culture carrier (Corning (registered trademark) Costar (registered trademark) cell culture 6-well plate, Thermo Fisher Scientific) in an amount of 6 × 10⁴ cells/well. The MSC culture medium without the activator was added to the cells in an amount of 1.3 mL/well, and cultivation was continued at 37°C for four days to prepare untreated MSCs (2D·activator-) having the passage number of 1.

To male APP/PS1 mice of 17-months old (Charles River Laboratories International, Inc.), the MSCs (2D·activator-) (2.5 × 10⁴ cells/mouse) or the MSCs (3D·activator+) (2.5 × 10⁴ cells/mouse) prepared in the above were each intrathecally administered, followed by keeping for 28 days. Twenty one days after the intrathecal administration, a Morris water maze test was performed for six days. In the Morris water maze test, each mouse was placed on the edge of a circular pool (a diameter of 1.2 m) filled with water (25°C ± 1°C), and the time for the mouse to reach a platform (a diameter of 10 cm) landable by a mouse foot, which was arranged right under the water surface, was measured. The platform was maintained at the same location, and the test was performed four times a day for five days while changing the location where the mouse was placed to evaluate the ability to learn the location of the platform (hidden platform tests). The day after the completion of the hidden platform tests, the platform was removed from the pool and a proportion of time in which the mouse swam within the quadrant region where the platform had been arranged in the previous hidden platform tests with respect to the total swimming time (60 seconds) was measured to evaluate memory ability of the mouse (probe test).

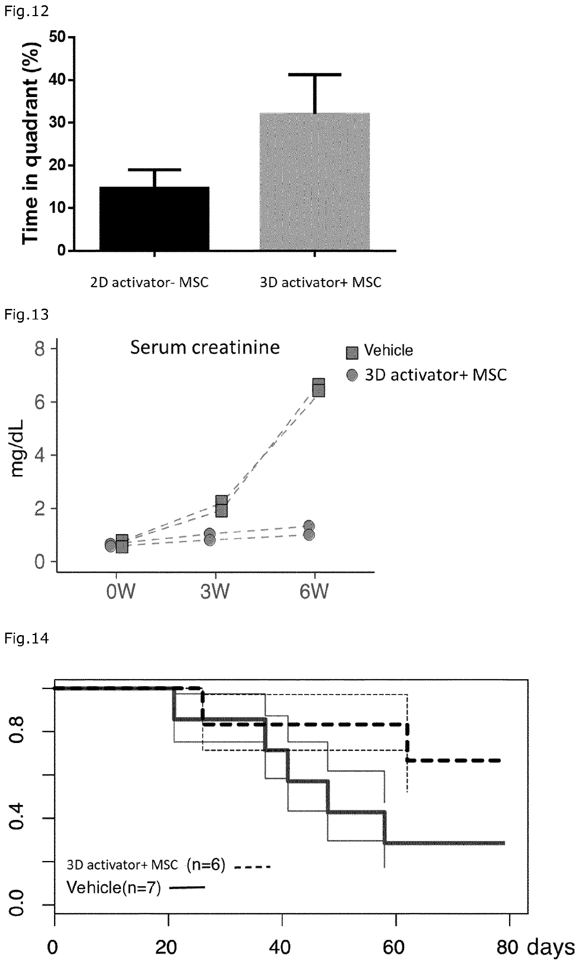

The learning test result and the memory test result using the Morris water maze are shown in Fig. 11 and Fig. 12, respectively. The mice to which the MSCs (3D·activator+) were administered had higher learning ability and memory ability as compared with the mice to which the MSCs (2D·activator-) were administered, confirming a high therapeutic effect of the MSCs (3D·activator+).

### Example 6 Therapeutic Effect of MSCs Activated on Three-Dimensional Culture Carrier (Diabetic Nephropathy)

To male OLETF rats of 14-months old (Hoshino Laboratory Animals, Inc.) having diabetic nephropathy, Rituximab (Chugai Pharmaceutical Co., Ltd.) was administered via the caudal vein in an amount of 5 mg/rat once a day for four days. From 10 to 14 days after the first administration of Rituximab, the MSC sheet prepared using the base material B in 3) of Example 2 was transplanted to the kidneys of the rats to which Rituximab was administered (3D activator+ MSC group, n = 6). The rats subjected to Rituximab administration alone were defined as Vehicle (Vehicle group, n = 7). Under isoflurane inhalation anesthesia, skin incision was performed under the lowest rib of the rats in a lateral position, and the muscle layer was incised to pull out one of the kidneys from the body. Gerota's fascia, fatty capsule, and fibrous capsule were carefully incised not to damage the adrenal gland, removed from the kidney surface, and pulled to the renal hilum. One MSC sheet having a size of 2.5 cm × 2.5 cm and another MSC sheet cut into a one-third portion were attached to the kidney so as to cover the whole kidney. The other kidney was also attached with the MSC sheets in the same manner. Bloods were collected from the rats on the day of the MSC sheet transplantation and 3 weeks and 6 weeks after the transplantation to measure serum creatinine using an enzymatic method (SRL, Inc.).

Fig. 13 shows the change in serum creatinine in the animals which survived 11 weeks after the transplantation (Vehicle group and 3D activator+ MSC group, n = 2 for each). In the Vehicle group, serum creatinine was increased over time, while, in the 3D activator+ MSC group, an increase in serum creatinine was not observed. Further, Fig. 14 shows the Kaplan-Meier curve representing survival rates until 11 weeks after the transplantation. The 3D activator+ MSC group showed a higher survival rates than the Vehicle group.

The rats used in the present study had not only diabetic nephropathy, but also an acute kidney disease induced by an anticancer treatment, and thus served as model animals exhibiting a severe chronic kidney disease in the same degree. The rats showed a very severe end-stage kidney disease corresponding to a nephropathy stage 4 (a kidney failure stage). It was confirmed that the sheet of the MSCs activated on the three-dimensional culture carrier showed a therapeutic effect to such a severe disease.

### Example 7 Expression Analysis of Therapeutic Effect Marker Gene in OA-MSCs Subjected to Activation Treatment

### 1) Activation Treatment of MSCs

MSCs derived from bone marrow collected from a patient with osteoarthritis of hip (n = 13) at the time of the artificial joint replacement surgery (OA-MSCs) were cultured in an MSC culture medium (DMEM containing 15% FBS, 1% penicillin, 1% streptomycin, and 100 mg/dL glucose) without the activator. The cells were recovered and seeded on Corning (registered trademark) Costar (registered trademark) cell culture 6-well plate (Thermo Fisher Scientific) in an amount of 6 × 10⁴ cells/well, and the MSC culture medium with or without the activator was added to the cells in an amount of 1.3 mL/well, followed by cultivation at 37°C for four days. By repeating this cultivation one more time, activation-treated OA-MSCs or untreated OA-MSCs having the passage number of 2 were prepared.

Further, MSCs derived from bone marrow collected from a 22-year old female having no symptom other than anterior cruciate ligament injury (MSCs derived from healthy person) were cultured and subjected to the activation treatment in the same manner as for the above OA-MSCs to prepare activation-treated MSCs derived from healthy person and untreated MSCs derived from healthy person.

### 2) Gene Expression Analysis

An expression level of each gene (OCT4, Nanog, SOX2, DNMT1, TERT, IL-6, IDO, TSG-6, p16^{ink4a}, RB, p21, p53, α-SMA, and 18s RNA) in each of the OA-MSCs prepared in the above 1) was measured by real-time PCR in the same manner as in 1) of Example 2.

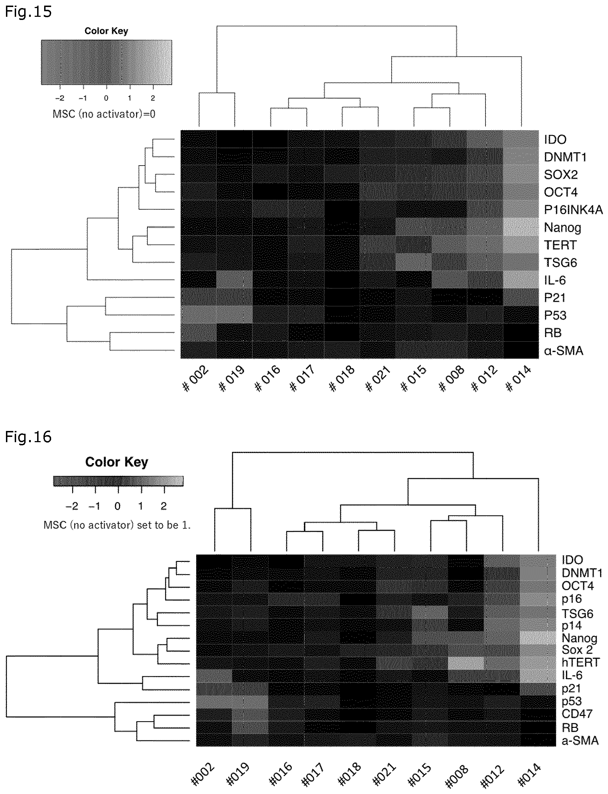

A ΔΔCT value of each gene expression in the activation-treated OA-MSCs was calculated on the basis of the untreated OA-MSCs using 18s RNA as a housekeeping gene, and gene expression profiles were analyzed and clustered. Fig. 15 shows the analysis result of the OA-MSCs in 10 cases.

Further, gene expression levels of p14^{ARF} and CD47 were measured by real-time PCR using primers having base sequences shown in Table 2 using the OA-MSCs in the same cases. The result was combined with the previous result of the gene expression levels and clustered. The result is shown in Fig. 16.

**[Table 2]**

| Gene | Base Sequence | SEQ ID NO: |
|---|---|---|
| P14ARF(F) | TACTGAGGAGCCAGCGTCTA | 29 |
| P14ARF (R) | TGCACGGGTCGGGTGAGAGT | 30 |
| CD47(F) | AGAAGGTGAAACGATCATCGAGC | 31 |
| CD47(R) | CTCA TCCAT ACCACCG GA TCT | 32 |

From these results, the gene expression profile of the MSCs differed in each patient as a separation source, making it clear that a response of the MSCs to the activation treatment differed depending on a patient from which the MSCs were separated.

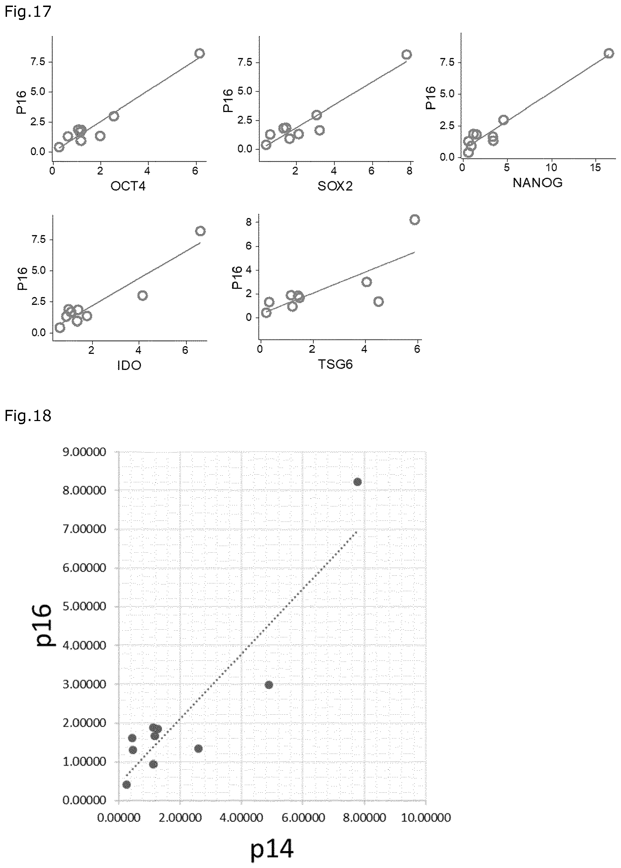

Further, unexpectedly, p16^{ink4a}, and p14^{ARF}, which had been considered to be a negative marker for a therapeutic effect of MSCs, belonged to the same cluster as that of DNMT1, Nanog, SOX2, OCT4, IDO, TSG6, and TERT, which were known to be a positive marker for a therapeutic effect of MSCs. Fig. 17 shows a correlation between relative expression levels of OCT4, SOX2, Nanog, IDO, and TSG6 genes and a relative expression level of p16^{ink4a} gene, and Fig. 18 shows a correlation between a relative expression level of p14^{ARF} gene and a relative expression level of p16^{ink4a} gene, in the OA-MSCs in 9 cases. High correlations were observed between OCT4, SOX2, Nanog, IDO, and TSG6 and p16^{ink4a}, and between p14^{ARF} and p16^{ink4a}.

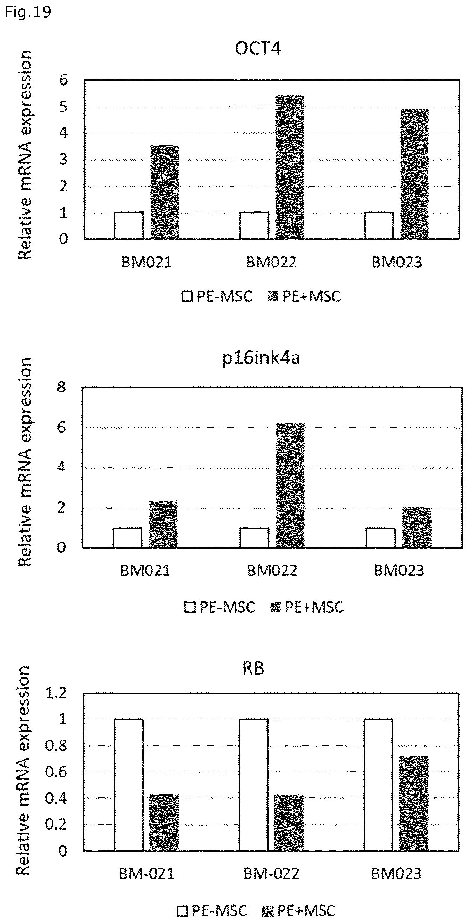

Further, gene expression of CDK4, CDK6, and RB, which were downstream factors of p16^{ink4a}, and CD47, which had been considered to be desirably expressed in cells used for a cell therapy, showed a tendency opposite to that of p16^{ink4a}. Fig. 19 shows relative expression levels of OCT4, p16^{ink4a}, and RB genes in the OA-MSCs (BM-021, BM-022, and BM-023) in 3 cases measured by real-time PCR. In each of the MSCs, expression of OCT4 and p16^{ink4a} was increased, while expression of RB was decreased by the activation treatment.

Total RNAs of the OA-MSCs in these three cases were subjected to a microarray analysis using Affymetrix Clariom (registered trademark) S Array. Data were analyzed using Affymetrix Transcriptome Analysis Console (TAC) Software. In each of the MSCs, an expression level of CDK6 was decreased about 2.04 times by the activation treatment (data not shown).

A tendency of expression changes in the above factors by the activation treatment was also observed in the MSCs from healthy person. For example, gene expression levels of SOX2 and p16^{ink4a} were increased more than 2 times by the activation treatment (data not shown). The above suggested that p16^{ink4a}, like the above known factors, could be used as a positive marker for a therapeutic effect of MSCs, while CDK4, CDK6, and RB could be used as a negative marker for a therapeutic effect.

### Example 8 Expression Analysis of Therapeutic Effect Marker Gene in OA-MSCs Subjected to Activation Treatment on the three-dimensional culture carrier

### 1) OCT4, SOX2, and p16 ^{ink4a}

A change in gene expression by the activation treatment on the three-dimensional culture carrier was analyzed using OA-MSCs. OA-MSCs were seeded on Preset VECELL 6-well (registered trademark) (Vessel Inc.) at 8 × 10⁴ cells/well, followed by cultivation using the MSC culture medium at 37°C for 72 hours. After removing the medium, an MSC culture medium containing the activator at 0.1 µg/mL or not containing the activator was added to the cells in an amount of 2 mL/well, followed by cultivation at 37°C for four days. Then, the cells were recovered to prepare activation-treated OA-MSCs and untreated OA-MSCs, having the passage number of 1. An expression level of each factor in these OA-MSCs was measured by real-time PCR.

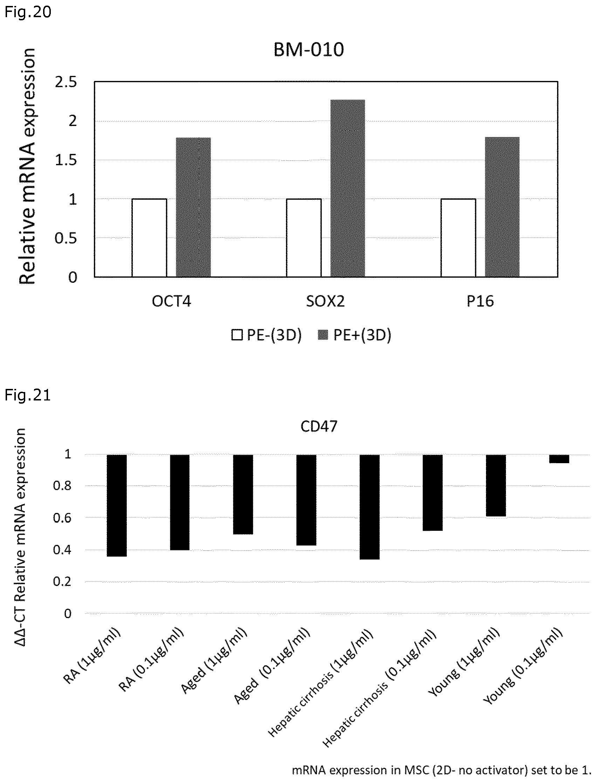

Fig. 20 shows relative expression levels of OCT4, SOX2, and p16^{ink4a} genes in the OA-MSCs (BM-010) in one case. It was confirmed that an expression level of each factor was also increased by the activation treatment on the three-dimensional culture carrier, as observed by the activation treatment on the two-dimensional culture carrier in Example 7.

### 2) CD47

Using MSCs derived from bone marrow collected from a patient with rheumatoid arthritis, a 72-year old male, a patient with hepatic cirrhosis, and a 38-year old male (RA, Aged, Hepatic cirrhosis, and Young, respectively), the activation treatment was performed on the three-dimensional culture carrier at two different activator concentrations (1 µg/mL and 0.1 µg/mL) in the same manner as in the above 1), thereby prepared activation-treated MSCs having the passage number of 1. Further, the same MSCs were seeded on a two-dimensional culture carrier (Corning (registered trademark) Costar (registered trademark) cell culture 6-well plate, Thermo Fisher Scientific) in an amount of 6 × 10⁴ cells/well. The MSC culture medium without the activator was added to the cells in an amount of 1.3 mL/well, and cultivation was continued at 37°C for four days to prepare untreated MSCs having the passage number of 1.

A gene expression level of CD47 in these MSCs was measured by real-time PCR. Fig. 21 shows a relative gene expression level of CD47 in the MSCs subjected to the activation treatment on the three-dimensional culture carrier with respect to that in the untreated MSCs. The gene expression level of CD47 was decreased by the activation treatment in each of the MSCs, and this tendency was more evident in the MSCs derived from the patient with rheumatoid arthritis, the 72-year old male, and the patient with hepatic cirrhosis. Considering that MSCs derived from a subject with a disease and an aged person generally have a lower therapeutic effect, it was suggested that CD47 could be used as a negative marker for a therapeutic effect.

### Example 9 Expression Analysis of Cell Surface Marker CD47

Using MSCs derived from bone marrow collected from a 38-year old male and a 72-year old male (MSCs from young person and MSCs from aged person, respectively), activation-treated MSCs and untreated MSCs having the passage number of 1 were prepared at an activator concentration of 0.1 µg/mL in the same manner as in 2) of Example 8. These MSCs were reacted with Zombie Violet (trademark) Dye (BioLegend, Inc.) for dead cell staining, then labelled with FITC anti-human CD47 antibodies (BioLegend, Inc.), and subjected to a flow cytometry analysis using FACSCanto (trademark) II (BD Biosciences). As a negative control, MSCs were reacted with isotype controls or only secondary antibodies and used. Data analysis was performed using FACSDiva (trademark) (BD Biosciences) and Kaluza V1.5a (Beckman Coulter, Inc).

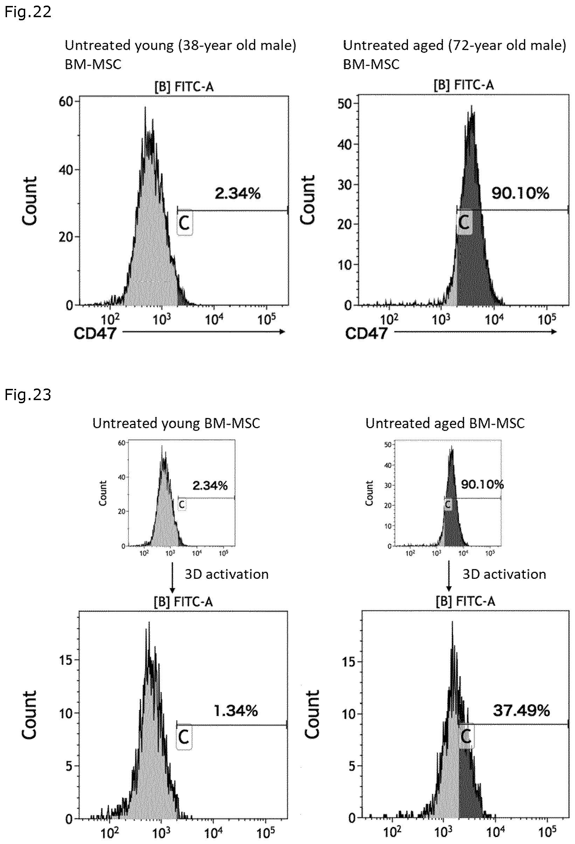

Fig. 22 shows the result of the untreated MSCs. MSCs having a signal intensity higher than that detected in the negative control was determined to be CD47 positive, while MSCs having a signal intensity lower than that detected in the negative control was determined to be CD47 negative. A CD47 positive cell proportion in the MSCs derived from young person, that is, a proportion of CD47 positive MSCs in the MSC population, showed a value as low as 2.34%, while a CD47 positive cell proportion in the MSCs derived from aged person showed a value as high as 90.10%.

Fig. 23 shows the result of the activation-treated MSCs. A CD47 positive cell proportion was decreased by the activation treatment both in the MSCs derived from young person and in the MSCs derived from aged person, in particular, the MSCs derived from aged person showed a significant reduction. Considering that the MSCs derived from aged person generally have a lower therapeutic effect on a disease, it was suggested that CD47 could be used as a negative marker for a therapeutic effect.

### Example 10 Function Evaluation of MSCs Highly Expressing p16^{ink4a} (Effect on Macrophage)

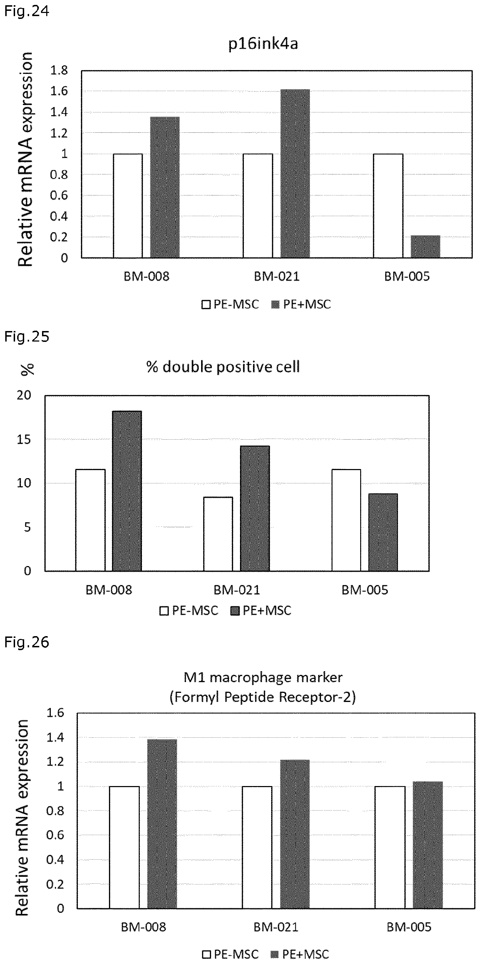

Among the OA-MSCs subjected to the activation treatment in 1) of Example 7, the OA-MSCs (BM-008, BM-021, and BM-005) in 3 cases having different degrees of expression changes were subjected to function evaluation. Fig. 24 shows a relative expression level of p16^{ink4a} gene in these OA-MSCs. An expression level of p16^{ink4a} was increased about 1.4 times in BM-008 and about 1.6 times in BM-021 by the activation treatment, while it was decreased about 0.2 times in BM-005.

The above three kinds of activation-treated OA-MSCs and their corresponding untreated OA-MSCs, a total six kinds of MSCs, were subjected to cocultivation with mouse macrophage-like cell line RAW 264.7. MSCs stained with PKH red dyes were seeded on a membrane portion of an insert of Cell Culture Insert (Falcon (registered trademark)) in an amount of 2 × 10⁴ cells and RAW 264.7 cells stained with PKH green dyes were seeded on a plastic plate portion in an amount of 2 × 10⁵ cells, followed by cocultivation using a DMEM medium at 37°C for 24 hours.

After completing the cocultivation, the MSCs were removed from the membrane of the insert using trypsin and transferred to the plastic plate portion where RAW 264.7 cells were present to perform direct contact cocultivation for 24 hours. After the direct contact cocultivation, cells were fixed by 4% paraformaldehyde and subjected to a flow cytometry analysis.

Fig. 25 shows a proportion of PKH red and PKH green double positive cells with respect to the total number of mononuclear cells excluding dead cells by the flow cytometry analysis. The double positive cells are considered to be cells corresponding to macrophages which have phagocytosed debris of dead cells derived from the MSCs, thus an increase in the number of these cells means an increase in phagocytic ability of the macrophages. By the activation treatment, a proportion of the double positive cells was increased in the samples using BM-008 and BM-021, while it was decreased in the sample using BM-005.

Further, RAW 264.7 cells were recovered after the cocultivation, and total RNAs were extracted using Tri Reagent (Molecular Research Center, Inc). Clone DNAs were synthesized by a reverse transcription reaction, and an expression level of Fpr2 (Formyl Peptide Receptor-2) as a marker for M1 macrophage was measured by real-time PCR using the following primer set.
Fpr(F): 5'-TCTACCATCTCCAGAGTTCTGTGG-3' (SEQ ID NO: 33)
Fpr(R): 5'-TTACATCTACCACAATGTGAACTA-3' (SEQ ID NO: 34)

Fig. 26 shows a relative expression level of Fpr in RAW 264.7 cells co-cultured with the OA-MSCs (BM-008, BM-021, and BM-005). By the activation treatment, an expression level of Fpr was increased in the samples using BM-008 and BM-021, while there was no change in the samples using BM-005.

In recent years, it is confirmed that chronic inflammation is involved in various diseases other than a disease conventionally considered as a chronic inflammatory disease. Examples of a disease in which chronic inflammation is involved include, in addition to a chronic inflammatory disease, an allergic disease, an autoimmune disease, cancer, an arteriosclerotic disease (ischemic heart disease, stroke, etc.), a neurodegenerative disease (Alzheimer's disease), a metabolic syndrome/lifestyle-related disease (obesity, diabetes, dyslipidemia, chronic kidney disease, non-alcoholic steatohepatitis, etc.), and a micturition disorder. Further, it is known that chronic inflammation is involved in aging.

In acute inflammation, M1 macrophages increase at the initial stage of inflammation and phagocytose various cells such as senescent cells that have served their purposes and damaged cells. It is believed that, after clearance via phagocytosis by M1 macrophages, M2 macrophages appear in the tissue and promote production of collagen or the like from surrounding cells by stimulation of cytokine or the like to repair the tissue. On the other hand, it is believed that, in chronic inflammation, an M2 macrophage-dominant state continues, so that various cells such as fibroblast continue to exist without being removed by phagocytosis, thus inflammation continues without the tissue repair, thereby facilitating tissue fibrosis (J. G. Tidbal et al., Nature Medicine, 2015, vol. 21, p. 665-666; X. M. Meng et al., Nature Reviews Nephrology, 2014, vol. 10, p. 493-503; A. Pellicoro et al., Nature Reviews Immunology, 2014, vol. 14, p. 181-194). It is speculated that induction of an M1 macrophage-dominant state is important to treat a disease in which chronic inflammation is involved, thus an increase in M1 macrophages can be used as an indicator for a therapeutic effect in these diseases in which chronic inflammation is involved.

In accordance with the study in Example 10, it was confirmed that the MSCs in which an expression level of p16^{ink4a} was increased by the activation treatment enhanced phagocytic ability of co-cultured macrophages and induced a shift to M1 type, however, the MSCs in which an expression level of p16^{ink4a} was decreased by the activation treatment did not show such an effect on macrophages. Since an increase in M1 macrophages could be used as an indicator for a therapeutic effect in the diseases in which chronic inflammation is involved, it was speculated that the MSCs in which an expression level of p16^{ink4a} was increased by the activation treatment was caused to enhance the therapeutic effect by the activation treatment, that is, activated, on the other hand, the MSCs in which an expression level of p16^{ink4a} was decreased by the activation treatment was not caused to enhance the therapeutic effect by the activation treatment, that is, not activated.

### Example 11 Function Evaluation of MSCs Highly Expressing p16^{ink4a} (Polymyositis)

### 1) Local Administration

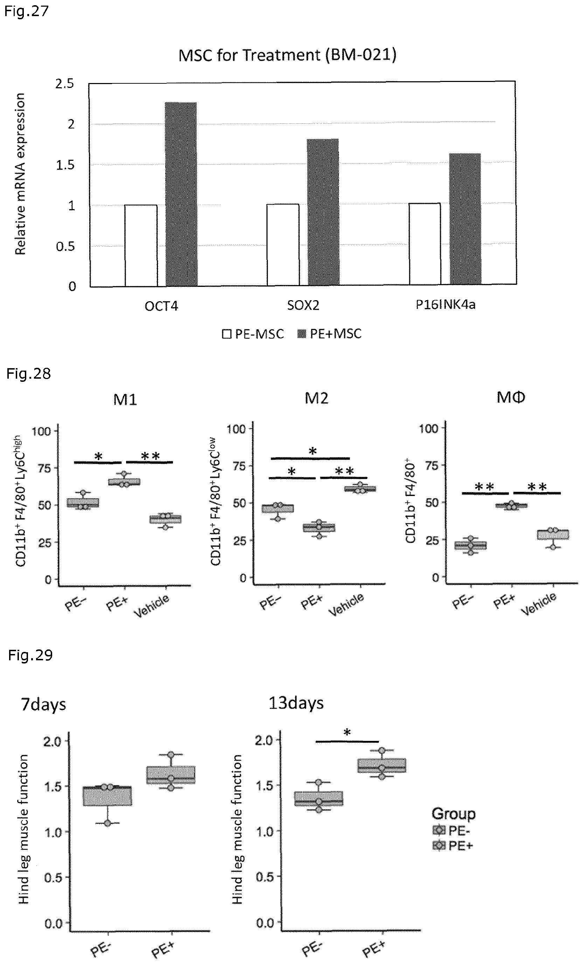

Using the OA-MSCs (BM-021) in which an expression level of p16^{ink4a} was increased by the activation treatment, a therapeutic effect on polymyositis, a disease in which chronic inflammation is involved, was examined. Fig. 27 shows relative expression levels of OCT4, SOX2, and p16^{ink4a} genes in BM-021. An expression level of each factor was increased by the activation treatment.

In a vicinity of the lymph node of each of 8-weeks old Balb/c mice, an emulsion composed of a myosin fraction (1 mg/mouse) prepared from a skeletal muscle of another Balb/c mouse and complete Freund's adjuvant (Chondrex, Inc., 200 pg/mouse) was administered. In the same time, pertussis toxin (List Biological Labs, Inc., 500 ng/mouse) was intraperitoneally administered to immunize the mice. One week and two weeks after the first immunization, the above emulsion was administered in the vicinity of the mouse lymph node for additional immunization to make polymyositis model mice. Four weeks after the first immunization, the mice were divided into 3 groups (n = 9), and the activation-treated MSCs (BM-021, 1 × 10⁵ cells/mouse), the untreated MSCs (BM-021, 1 × 10⁵ cells/mouse), prepared in Example 7, or PBS (vehicle) were locally administered to the triceps surae muscle of the hind leg.

Two days after the MSC administration, the triceps surae muscles were collected and subjected to a collagenase enzyme treatment. Then, red blood cells were removed using RBC Lysis buffer to make a single-cell suspension. Subsequently, an antigen-antibody reaction was performed using a dead cell marker (Zonbi Dye), PE-Cy7-CD11b antibodies, APC-F4/80 antibodies, and FITC-Ly6C antibodies, followed by a flow cytometry analysis. The result is shown in Fig. 28. Proportions of the total macrophages and M1 macrophages were significantly increased in the mice (PE+) to which the activation-treated MSCs were administered as compared with the mice (PE-) to which the untreated MSCs were administered.

Seven days and 13 days after the MSC administration, the following function evaluation of the hind leg muscle was performed. Each mouse was held by the neck and the tail and allowed to grasp a metal wire having a diameter of 1 mm by the hind legs. After visually confirming that the mouse grasped the metal wire by the both hind legs, the wire was pulled away from the mouse until the mouse could not grasp the metal wire any more. A pulling force (gram) at the time when the mouse could not grasp the wire was recorded as hind leg muscle strength. The pulling force was measured by an electronic balance having a resolution of 0.01 g (A & D Company, Ltd.). Measurement was performed three times for each mouse and the median of these measurements was used as a representative value. The result is shown in Fig. 29. The hind leg muscle function was more enhanced in the PE+ mice than in the PE- mice.

### 2) Systemic Administration

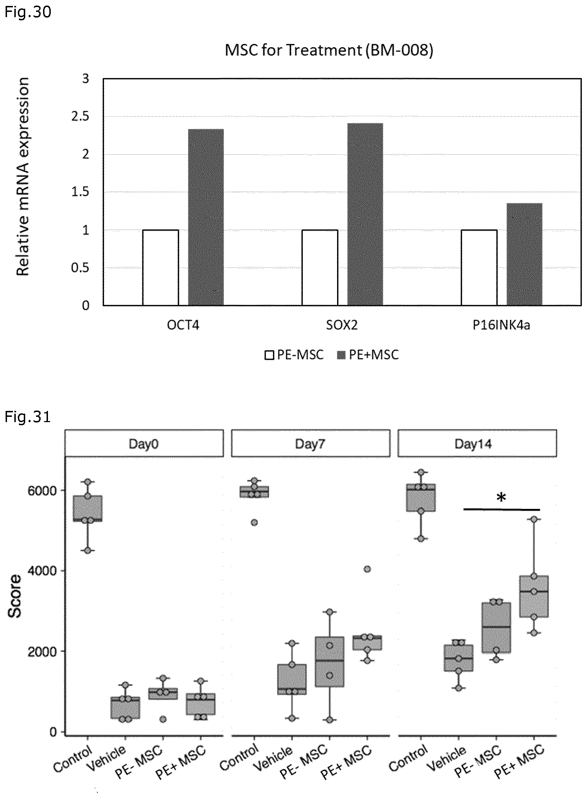

Next, using the OA-MSCs (BM-008) in which an expression level of p16^{ink4a} was increased by the activation treatment, a therapeutic effect on polymyositis was examined. Fig. 30 shows relative expression levels of OCT4, SOX2, and p16^{ink4a} genes in BM-008. An expression level of each factor was increased by the activation treatment, like BM-021.

Polymyositis model mice was made in the same manner as in the above 1), and, four weeks after the first immunization, the activation-treated MSCs (BM-008, 1 × 10⁶ cells/mouse), the untreated MSCs (BM-008, 1 × 10⁶ cells/mouse), prepared in Example 7, or PBS (vehicle) were intravenously administered to the mice. Zero, seven, and 14 days after the MSC administration, muscle function was evaluated by an inverted screen test as described below. Each mouse was placed on a metal mesh having a diameter of 1 mm and a mesh size of 5 mm and the metal mesh was turned upside down to measure the time that mouse could hang on the metal mesh by the forelimbs and the hind legs. The product of the weight (gram) of the mouse and the hanging time (second) was used as muscle function data. The muscle function was more enhanced in the mice (PE+ MSC) to which the activation-treated MSCs were administered than in the mice (PE- MSC) to which the untreated MSCs were administered (Fig. 31).

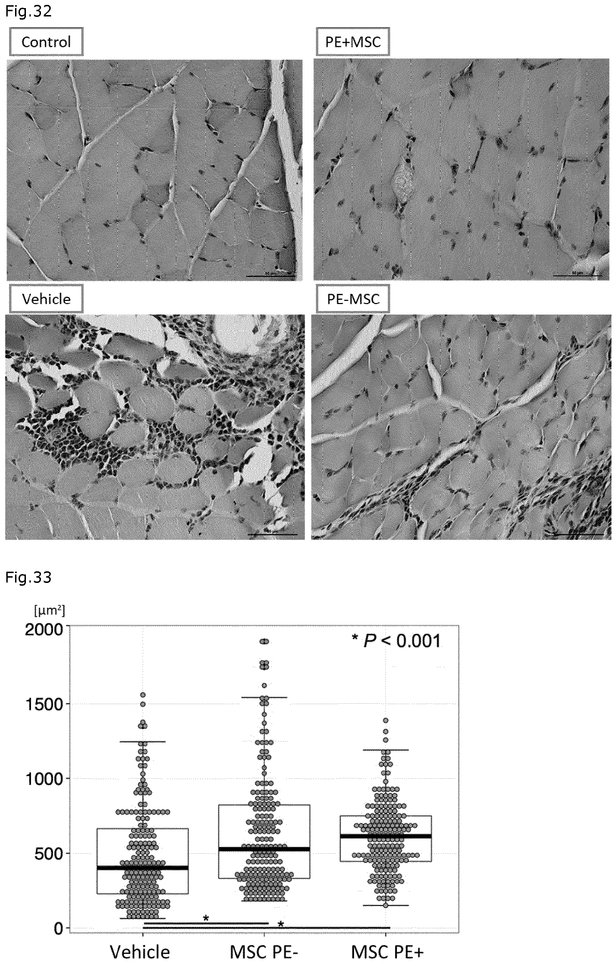

Fig. 32 shows an example of HE staining images of the mouse muscle tissues collected 14 days after the MSC administration. There was less infiltration of inflammatory cells into the muscle tissues in the PE+ MSC mice than in the PE- MSC mice. Further, muscle cross-sectional areas were analyzed using the mouse muscle tissues (n = 3 for each group) collected 14 days after the MSC administration. For analyzing the muscle cross-sectional area, a frozen section of 8 µm was created and subjected to staining using rabbit anti-laminin antibodies (Abcam plc.) as a primary antibody and anti-rabbit Cy3-labeled antibodies as a secondary antibody. Image data were acquired using a confocal microscope (Nikon Corp.). Laminin detects the basement membrane of muscle, thus a closed region surrounded by laminin represents a muscle cross-sectional area. An area of the closed region was calculated from the image data using ImageJ. The mice (MSC PE+) to which the activation-treated MSCs were administered had the larger muscle cross-sectional areas than the mice (MSC PE-) to which the untreated MSCs were administered (Fig. 33).

As shown in 1) and 2) described above, it was confirmed that the MSCs in which an expression level of p16^{ink4a} was increased by the activation treatment increased proportions of the total macrophages and M1 macrophages more and facilitated a repair of the tissue damaged by inflammation more as compared with the untreated MSCs. This indicates that an increase in expression of p16^{ink4a} gene in the MSCs is an event that has a correlation with the activation of the MSCs, that is, with the enhancement in a therapeutic effect of the MSCs.

### Example 12 Function Evaluation of MSCs Highly Expressing p16^{ink4a} (Skin Defect)

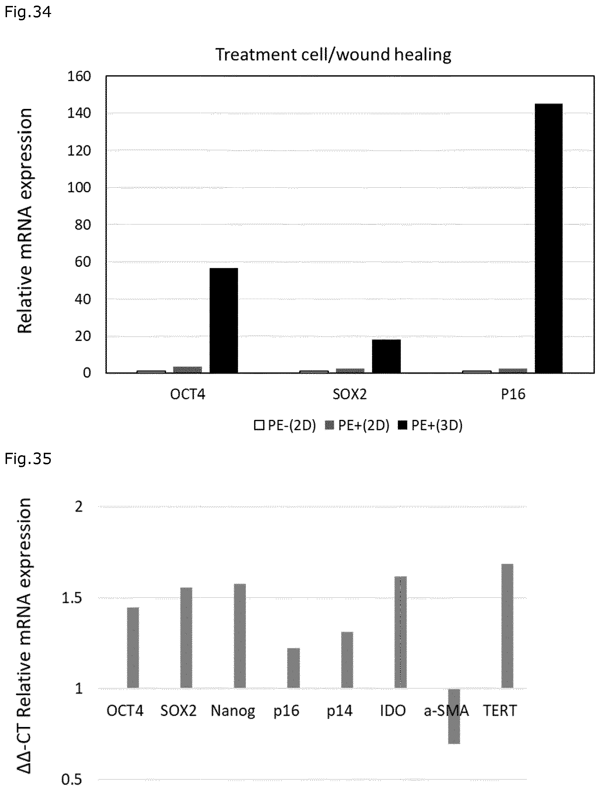

Expression levels of OCT4, SOX2, and p16^{ink4a} genes in the three kinds of MSCs administered to the rats in Example 4 were measured by real-time PCR. Fig. 34 shows relative expression levels with respect to those in the MSCs (PE-(2D)) cultured in the absence of the activator on the two-dimensional culture carrier. An expression level of each factor was increased by the activation treatment, in particular, a significant increase was observed when the three-dimensional culture carrier was used. From a tendency of the wound healing effect confirmed in Example 4, it was confirmed that the MSCs in which an expression level of p16^{ink4a} was increased more quickly healed the wound as compared with the untreated MSCs, in particular, the MSCs in which an expression level of p16^{ink4a} was significantly increased had very high wound healing capability with a small number of cells.

### Example 13 Function Evaluation of MSCs Highly Expressing p16^{ink4a} (Diabetic Nephropathy)

Expression levels of OCT4, SOX2, Nanog, p16^{ink4a}, p14^{ARF}, IDO, α-SMA, and TERT genes in the MSCs administered to the rat in Example 6 were measured by real-time PCR. Fig. 35 shows relative expression levels with respect to those in the MSCs cultured in the absence of the activator on the two-dimensional culture carrier. Expression levels of OCT4, SOX2, Nanog, p16^{ink4a}, p14^{ARF}, IDO, and TERT were increased, while an expression level of α-SMA was decreased by the activation treatment on the three-dimensional culture carrier. From the treating effect for nephropathy confirmed in Example 6, it was confirmed that the MSCs in which expression levels of p16^{ink4a} and p14^{ARF} were increased exhibited the high treating effect for nephropathy.

### Example 14 Function Analysis of p16^{ink4a}-Deficient MSCs (Polymyositis)

### 1) Preparation of p16^{ink4a}-knockout MSCs

CDKN2A gene encoding p16^{ink4a} and p14^{ARF} in MSCs derived from bone marrow collected from OA-MSCs was knocked out using p14 ARF/p16 CRISPR/Cas9 KO plasmids (Santa Cruz Biotechnology, Inc.). A CRISPR/Cas9 knockout plasmid in which a target gRNA for CDKN2A gene knockout was integrated or a scramble plasmid in which a scramble gRNA was integrated instead of the target gRNA as a control was transfected into the MSCs. These MSCs were subjected to the activation treatment in the same manner as in 2) of Example 8 and then subjected to immunostaining by reacting the MSCs with anti-P16^{ink4a} antibodies (anti-p16INK4A human, Proteintech Group, Inc.) as a primary antibody and labeling the MSCs with Alxa 647 anti-rabbit IgG (Jackson ImmunoResearch Inc.) as a secondary antibody.

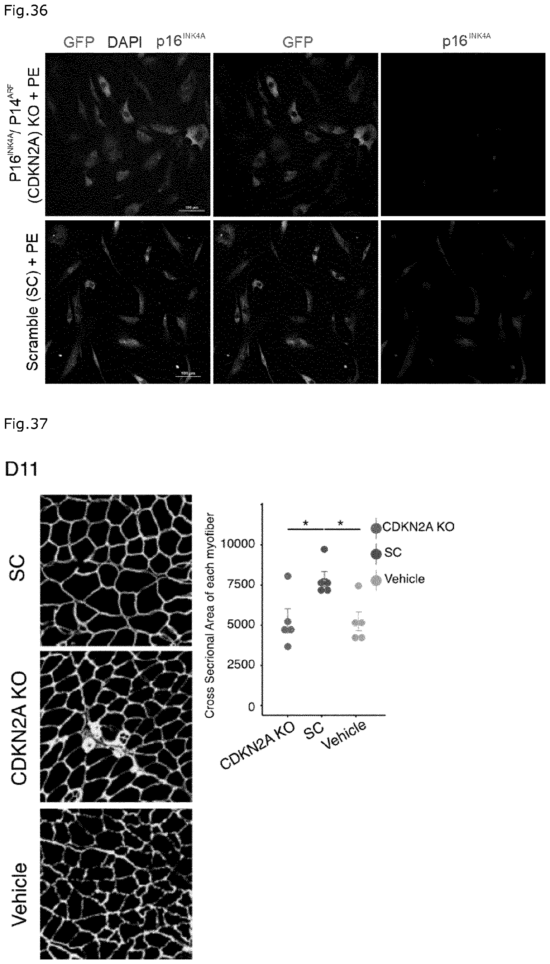

Fig. 36 shows fluorescent observation images of the MSCs. Fluorescence of p16^{ink4a} was not observed only in the MSCs to which the knockout plasmid was introduced (knockout MSC), confirming that p16^{ink4a} was knocked out in the MSCs.

### 2) Therapeutic Effect of MSCs in Polymyositis Model Mouse

The activation-treated knockout MSCs, the scramble MSCs, described in the above 1), or PBS were each locally administered to the triceps surae muscle of the polymyositis model mice in the same manner as in 1) of Example 11 to evaluate a therapeutic effect. Fig. 37 shows an example of laminin staining images of the mouse muscle tissues collected 11 days after the MSC administration and an analysis result of the muscle cross-sectional areas. The muscle cross-sectional areas were increased in the mice to which the scramble MSCs were administered (SC) as compared with the mice to which PBS was administered (Vehicle), however, an increase in the muscle cross-sectional areas were not observed in the mice to which the knockout MSCs were administered (CDKN2A KO). This confirmed that the therapeutic effect was reduced in the MSCs in which p16^{ink4a} was knocked out, thereby showing that p16^{ink4a} could be a positive marker for the therapeutic effect.

### Example 15 Function Evaluation of MSCs Highly Expressing CD47 (Skin Defect)

### 1) Preparation of MSCs Highly Expressing CD47

By using CD47 CRISPR activation plasmid (Santa Cruz Biotechnology, Inc.), a plasmid in which a target gRNA for activating CD47 was integrated was transfected into MSCs derived from bone marrow collected from a 38-year old male to make MSCs over-expressing CD47.

### 2) Therapeutic Effect of MSCs on Skin Defect Model Mouse

Skin defect model rats were made by excising a 1.5 cm × 1.5 cm skin in two places on the back of 35-weeks old Balb/c mice. One day after the skin excision, the MSCs (2.5 × 10⁴ cells/mouse) prepared in the above 1), control MSCs (2.5 × 10⁴ cells/mouse) in which the plasmid was not transfected, or PBS (Vehicle) were intravenously administered to the mice, followed by keeping for nine days.

Fig, 38 shows an amount of changes in wound areas from Day 3 to Day 9 after the skin excision and images of skin defect parts photographed on Day 9. It was confirmed that the mice to which CD47 over-expressing MSCs were administered (CD47activate) had a smaller amount of changes in the wound areas than the mice to which the control MSCs are administered (Control), that is, wound healing was delayed, showing that CD47 could be a negative marker for a therapeutic effect.

### Example 16 Function Evaluation of MSCs Having Different Expression Levels of CD47 (Cognitive Function)

### 1) Preparation of MSCs Having Different Expression Levels of CD47

MSCs derived from bone marrow collected from OA-MSCs were subjected to the activation treatment on the three-dimensional culture carrier at an activator concentration of 0.0001 mg/mL in the same manner as described in 2) of Example 8 to prepare activation-treated MSCs having the passage number of 1. Further, the same MSCs were seeded on a two-dimensional culture carrier (Corning (registered trademark) Costar (registered trademark) cell culture 6-well plate, Thermo Fisher Scientific) in an amount of 6 × 10⁴ cells/well. The MSC culture medium without the activator was added to the cells in an amount of 1.3 mL/well, and cultivation was continued at 37°C for four days to prepare untreated MSCs having the passage number of 1. A CD47 positive cell proportion in these MSCs were measured as in Example 9 to find that the CD47 positive cell proportion of the activation-treated MSCs was less than 20%, while the CD47 positive cell proportion of the untreated MSCs was more than 90%.

### 2) Therapeutic Effect of MSCs in Alzheimer's disease Model Mouse

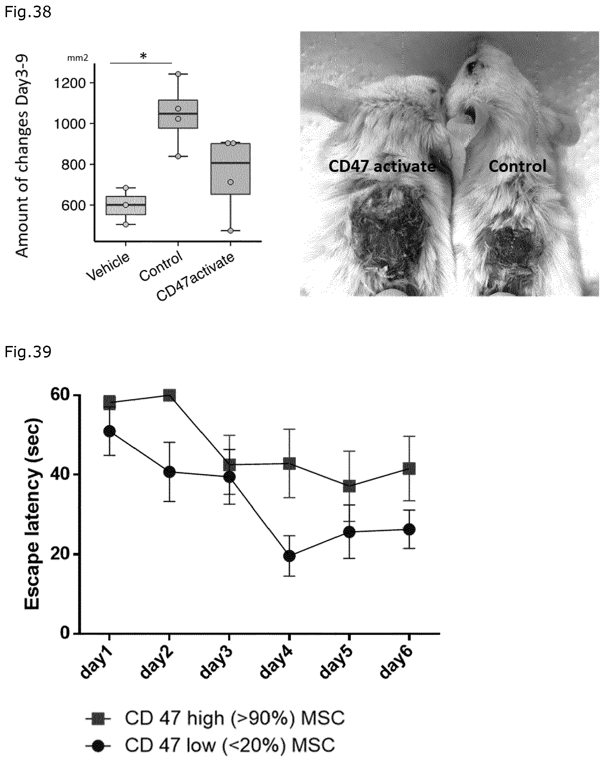

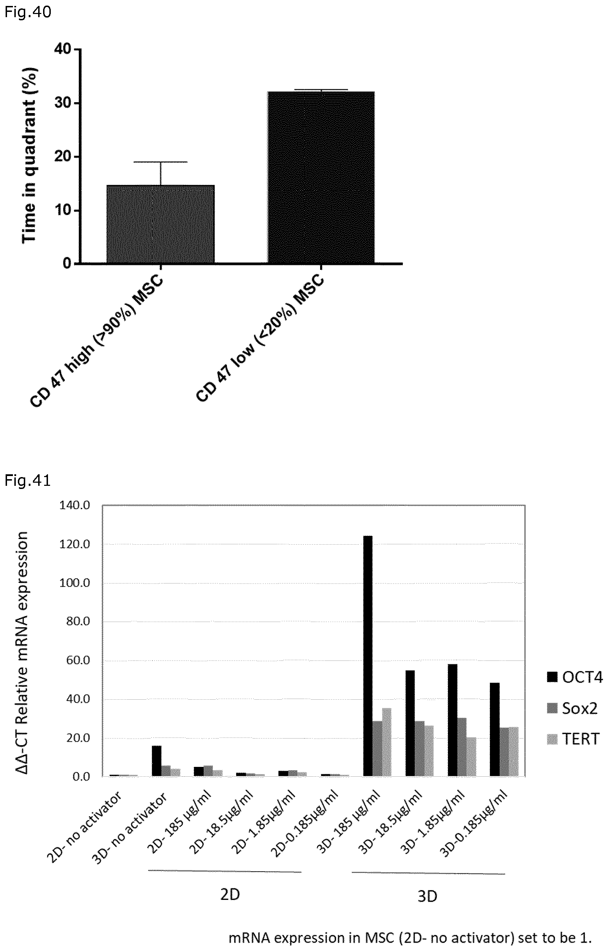

Two kinds of MSCs in the above 1) were each intrathecally administered to APP/PS1 mice as in Example 5 to evaluate the therapeutic effect. The learning test result and the memory test result using the Morris water maze are shown in Fig. 39 and Fig. 40, respectively. The mice to which the MSCs having a high CD47 positive cell proportion were administered had lower learning ability and memory ability than the mice to which the MSCs having a low CD47 positive cell proportion were administered, showing that CD47 could be a negative marker for a therapeutic effect.

### Example 17 Activation Treatment of MSCs Using Activators by Different Extraction Methods

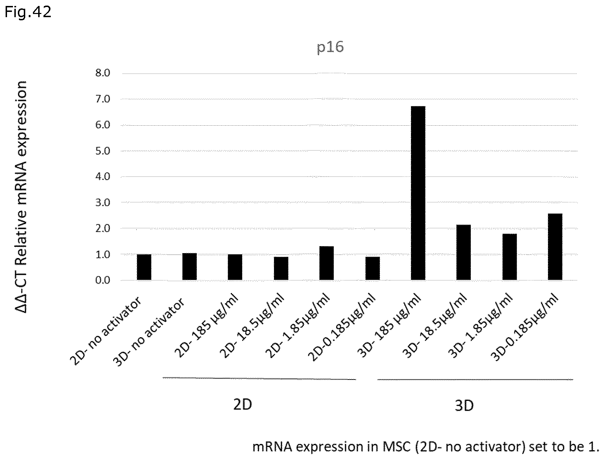

An activator was prepared by concentrating a commercially available human placenta extract ("Melsmon", MELSMON Pharmaceutical Co., Ltd.) using an evaporator, and an activation treatment of OA-MSCs was performed using this extract on Preset VECELL and a two-dimensional culture carrier as in 2) of Example 8. An expression level of each gene in these OA-MSCs was measured by real-time PCR. Fig. 41 and Fig. 42 show relative gene expression levels of OCT4, SOX2, TERT, and p16^{ink4a} in each of the MSCs with respect to relative gene expression levels of those genes in the MSCs (2D·no activator) cultured in the absence of the activator on the two-dimensional culture carrier. The activator prepared from the human placenta extract showed the stronger activation effect on the three-dimensional culture carrier, the same as the activator prepared in 1) of Example 1.

## Claims

1. A method for producing activated mesenchymal stem cells, comprising an activation step of treating mesenchymal stem cells by an activator that includes an extract from a mammalian fetal appendage as an active ingredient, wherein, in the activation step, the mesenchymal stem cells are cultured in a medium containing the activator using a cell culture carrier having a three-dimensional structure formed of a fiber.

2. The method according to claim 1, wherein the cell culture carrier has openings formed of the fiber having an average fiber diameter of from nanometer order to micrometer order on the cell contacting surface.

3. The method according to claim 2, wherein an average diameter of the openings is from 500 nm to 1000 µm.

4. The method according to any one of claims 1 to 3, wherein the cell culture carrier is formed by including a nanofiber made of a biodegradable polymer.

5. The method according to any one of claims 1 to 4, wherein a concentration of the activator in the medium is from 1/10 to 1/100000 of a concentration of the activator in the medium in the activation step of culturing mesenchymal stem cells using a cell culture carrier having no three-dimensional structure formed of the fiber.

6. The method according to any one of claims 1 to 5, wherein the concentration of the activator in the medium is from 0.01 µg/mL to 500 µg/mL in terms of protein.

7. The method according to any one of claims 1 to 6, wherein the cells are passaged 2 times or less in the activation step.

8. The method according to any one of claims 1 to 7, wherein the mesenchymal stem cells are derived from bone marrow.

9. The method according to any one of claims 1 to 8, wherein the mesenchymal stem cells are separated from a subject with a disease.

10. A cell preparation including mesenchymal stem cells having a CD47 positive cell proportion of 2% or less.

11. The cell preparation according to claim 10, wherein the mesenchymal stem cells are the activated mesenchymal stem cells produced by the method according to any one of claims 1 to 9.

12. A method for producing a cell preparation including mesenchymal stem cells, comprising a step of concentrating CD47 negative mesenchymal stem cells from a mesenchymal stem cell population.

13. A marker for evaluating a therapeutic effect of mesenchymal stem cells, the marker being selected from the group consisting of p16^{ink4a} and p14^{ARF} and having a positive correlation with the therapeutic effect of the mesenchymal stem cells.

14. A marker for evaluating a therapeutic effect of mesenchymal stem cells, the marker being selected from the group consisting of CDK4, CDK6, RB, and CD47 and having a negative correlation with the therapeutic effect of the mesenchymal stem cells.

15. A method for determining a therapeutic effect of mesenchymal stem cells, comprising: a measurement step of measuring a gene or protein expression level of at least one selected from the group consisting of p16^{ink4a}, p14^{ARF}, CDK4, CDK6, RB, and CD47 in test mesenchymal stem cells; a comparison step of comparing the measured gene or protein expression level with a gene or protein expression level in control mesenchymal stem cells; and a determination step of determining the test mesenchymal stem cells to have a higher therapeutic effect than the control mesenchymal stem cells if the gene or protein expression level of at least one selected from the group consisting of p16^{ink4a} and p14^{ARF} is higher in the test mesenchymal stem cells than in the control mesenchymal stem cells and/or if the gene or protein expression level of at least one selected from the group consisting of CDK4, CDK6, RB, and CD47 is lower in the test mesenchymal stem cells than in the control mesenchymal stem cells.

16. The method according to claim 15, wherein
the measurement step further includes measurement, in the test mesenchymal stem cells, of a gene or protein expression level of:
(A) at least one selected from the group consisting of DNMT1, Nanog, SOX2, OCT4, IDO, TSG6, IL-6, and TERT; and/or
(B) at least one selected from the group consisting of p53 and α-SMA, and
the determination step determines that test mesenchymal stem cells have a higher therapeutic effect than the control mesenchymal stem cells if the gene or protein expression level of (A) is higher in the test mesenchymal stem cells than in the control mesenchymal stem cells and/or if the gene or protein expression level of (B) is lower in the test mesenchymal stem cells than in the control mesenchymal stem cells, in addition to the higher gene or protein expression level of at least one selected from the group consisting of p16^{ink4a} and p14^{ARF} in the test mesenchymal stem cells than in the control mesenchymal stem cells and/or the lower gene or protein expression level of at least one selected from the group consisting of CDK4, CDK6, RB, and CD47 in the test mesenchymal stem cells than in the control mesenchymal stem cells.

17. The method according to claim 15 or 16, wherein, in the measurement step, a proportion of CD47 positive cells in the mesenchymal stem cells is measured instead of the gene or protein expression level of CD47, and, in the determination step, the test mesenchymal stem cells are determined to have the higher therapeutic effect than the control mesenchymal stem cells if the proportion of CD47 positive cells is lower in the test mesenchymal stem cells than in the control mesenchymal stem cells instead of the lower gene or protein expression level of CD47 in the test mesenchymal stem cells than in the control mesenchymal stem cells.

18. The method according to any one of claims 15 to 17, wherein the test mesenchymal stem cells are mesenchymal stem cells cultured in a medium including an activator containing an extract from a mammalian fetal appendage as an active ingredient.

19. The method according to any one of claims 15 to 18, wherein the test mesenchymal stem cells are mesenchymal stem cells cultured using a cell culture carrier having a three-dimensional structure formed of a fiber.

20. The method according to any one of claims 15 to 19, wherein the test mesenchymal stem cells are mesenchymal stem cells separated from a subject being to undergo an autologous transplantation therapy of the mesenchymal stem cells.

21. The method according to any one of claims 15 to 20, wherein the test mesenchymal stem cells are derived from bone marrow.

22. A method for determining suitability of mesenchymal stem cells to be treated with a treatment for enhancing a therapeutic effect of mesenchymal stem cells, comprising: a measurement step of measuring a gene or protein expression level of at least one selected from the group consisting of p16^{ink4a}, p14^{ARF}, CDK4, CDK6, RB, and CD47 in treated mesenchymal stem cells; a comparison step of comparing the measured gene or protein expression level with a gene or protein expression level in untreated mesenchymal stem cells; and a determination step of determining the mesenchymal stem cells to be suitable to be treated with the treatment if the gene or protein expression level of at least one selected from the group consisting of p16^{ink4a} and p14^{ARF} is higher in the treated mesenchymal stem cells than in the untreated mesenchymal stem cells and/or if the gene or protein expression level of at least one selected from the group consisting of CDK4, CDK6, RB, and CD47 is lower in the treated mesenchymal stem cells than in the untreated mesenchymal stem cells.

23. The method according to claim 22, wherein
the measurement step further includes measurement, in each of the treated mesenchymal stem cells and the untreated mesenchymal stem cells, of a gene or protein expression level of:
(A) at least one selected from the group consisting of DNMT1, Nanog, SOX2, OCT4, IDO, TSG6, IL-6, and TERT; and/or
(B) at least one selected from the group consisting of p53 and α-SMA, and
the determination step determines that the mesenchymal stem cells are suitable to be treated with the treatment if the gene or protein expression level of (A) is higher in the treated mesenchymal stem cells than in the untreated mesenchymal stem cells and/or if the gene or protein expression level of (B) is lower in the treated mesenchymal stem cells than in the untreated mesenchymal stem cells, in addition to the higher gene or protein expression level of at least one selected from the group consisting of p16^{ink4a} and p14^{ARF} in the treated mesenchymal stem cells than in the untreated mesenchymal stem cells and/or the lower gene or protein expression level of at least one selected from the group consisting of CDK4, CDK6, RB, and CD47 in the treated mesenchymal stem cells than in the untreated mesenchymal stem cells.

24. The method according to claim 22 or 23, wherein, in the measurement step, a proportion of CD47 positive cells in the mesenchymal stem cells is measured instead of the gene or protein expression level of CD47, and, in the determination step, the mesenchymal stem cells are determined to be suitable to be treated with the treatment if the proportion of CD47 positive cells is lower in the treated mesenchymal stem cells than in the untreated mesenchymal stem cells instead of the lower gene or protein expression level of CD47 in the treated mesenchymal stem cells than in the untreated mesenchymal stem cells.

25. The method according to any one of claims 22 to 24, wherein the treatment is culturing the mesenchymal stem cells in a medium including an activator containing an extract from a mammalian fetal appendage as an active ingredient.

26. The method according to any one of claims 22 to 25, wherein the treatment is culturing the mesenchymal stem cells using a cell culture carrier having a three-dimensional structure formed of a fiber.

27. The method according to any one of claims 22 to 26, wherein the mesenchymal stem cells are mesenchymal stem cell separated from a subject being to undergo an autologous transplantation therapy of the mesenchymal stem cells.

28. The method according to any one of claims 22 to 27, wherein the mesenchymal stem cells are derived from bone marrow.
